# EUROPEAN PATENT SPECIFICATION

(11) **EP 2 814 945 B1**
(45) Date of publication and mention of the grant of the patent: **02.08.2017**
(21) Application number: 13701624.2
(22) Date of filing: 28.01.2013
(51) Int. Cl.: C12N 1/20, C12P 13/12, C12P 13/22, C12P 1/00

(54) **A CELL WITH REDUCED PPGPPASE ACTIVITY**
ZELLE MIT REDUZIERTER PPGPPASE-AKTIVITÄT
CELLULE DOTÉE D'UNE ACTIVITÉ PPGPPASE MOINDRE

(30) Priority: 17.02.2012 EP 12156052
(43) Date of publication of application: 24.12.2014
(73) Proprietor: Evonik Degussa GmbH, 45128 Essen (DE)
(72) Inventor: SCHNEIDER, Frank, 33790 Halle (DE); GERTH, Caroline, 33813 Oerlinghausen (DE)
(86) International application number: PCT/EP2013/051529
(87) International publication number: WO 2013/120685

(56) References cited:
- EP-A1- 2 204 441
- US-B1- 6 610 836
- GENTRY DANIEL R ET AL: "Mutational analysis of the Escherichia coli spoT gene identifies distinct but overlapping regions involved in ppGpp synthesis and degradation", MOLECULAR MICROBIOLOGY, WILEY-BLACKWELL PUBLISHING LTD, GB, vol. 19, no. 6, 1 January 1996 (1996-01-01), pages 1373-1384, XP008090292, ISSN: 0950-382X, DOI: 10.1111/J.1365-2958.1996.TB02480.X

## Description

The present invention relates to an *E. coli* cell which is genetically modified over its wild type in such a way that it has a reduced ppGppase activity relative to its wild type and overproduces preferably an essential amino acid, even more preferably an essential amino acid derived from serine, most preferably methionine or tryptophan, and to a method of preparing an essential amino acid thereof derived from serine, comprising the steps of a) culturing the cell according to the first aspect of the present invention or to any of its embodiments, and b) optionally: purifying the amino acid.

Sulphur-containing and aromatic L-amino acids are of great economic importance. L-Cysteine is used as a food additive, as a starting substance for pharmacological active compounds (e.g. N-acetylcysteine) and for cosmetics. The amino acids L-methionine and L-tryptophan play a very prominent role in animal nutrition. They belong to the essential amino acids which cannot be produced by biosynthesis in the metabolism of vertebrates. In animal breeding it must consequently be ensured that sufficient amounts of the particular amino acid are taken in with the feed. However, since L-methionine for example is often present in conventional feedstuff plants (such as soya or cereals) in amounts which are too low to ensure optimum animal nutrition, particularly for pigs and poultry, it is advantageous to add methionine as an additive to the animal feed. D-Methionine can be converted into biologically active L-methionine by vertebrates. A racemate of D- and L-methionine is therefore usually added to the animal feed. In animals L-homocysteine can be converted by transmethylation into and thus replace L-methionine.

In the prior art, amino acids such as methionine are prepared by chemical synthesis. This involves first reacting acrolein and methylmercaptan to give 3-methylthiopropionaldehyde, which in turn with cyanide, ammonia and carbon monoxide results in hydantoin. The latter may finally be hydrolysed to give the racemate, an equimolar mixture of the two stereoisomers, D- and L-methionine. Since only the L form constitutes the biologically active form of the molecule, the D form present in the feed must first be converted metabolically by de- and transamination into the active L form.

In contrast to methionine, most other natural, proteinogenic amino acids, such as -tryptophan for example, are chiefly prepared by fermentation of microorganisms, taking advantage of the fact that microorganisms have appropriate biosynthetic pathways for synthesizing the natural amino acids. In addition, many fermentation processes achieve very favourable production costs with inexpensive reactants such as glucose and mineral salts, and moreover deliver the biologically active L form of the particular amino acid.

Amino acids of this type are known to be produced by fermentation of *Enterobacteriaceae* strains, in particular *Escherichia coli* (*E. coli*) and *Serratia marcescens*. Because of the great importance, work is constantly being undertaken to improve the preparation processes. Improvements to the processes can relate to fermentation measures, such as stirring and supply of oxygen for example, or the composition of nutrient media, for example selection of the sugar used and the sugar concentration during fermentation, or to the working-up to the product form, for example by ion exchange chromatography, or to the intrinsic output properties of the microorganism itself.

Biosynthetic pathways of amino acids are subject to strict metabolic control in wild-type strains, which ensures that the amino acids are produced only to the extent of the cell's own requirement. An important prerequisite for efficient production processes is therefore the availability of suitable microorganisms which, in contrast to wild-type organisms, have a drastically increased production output in excess of their own requirements (overproduction) for the preparation of the desired amino acid.

Such amino acid-overproducing microorganisms may be generated by conventional mutation/selection processes and/or by modern, specific, recombinant techniques (metabolic engineering). The latter case involves firstly identifying genes or alleles which effect an amino acid overproduction by their modification, activation or inactivation. Molecular biology techniques are then used to introduce these genes/alleles into a microorganism strain or inactivate them, so as to optimize overproduction. However, often only the combination of several different measures results in a truly efficient production. Said genes or alleles may code for enzymes, transcription factors, transporters and other polypeptides, cofactors or cofactor-synthesizing polypeptides or components capable of influencing the synthesis of the amino acid of interest. In a preferred embodiment, the term "components", as understood herein, means a subunit of such a polypeptide influencing the synthesis of the amino acid of interest, which subunit, although smaller than the whole system encoded by the gene or operon, for example a subunit or a domain of a polypeptide, is functional with regard to the task of the whole system.

L-Methionine derives from aspartate and serine. In *E. coli* sulphur is introduced by way of L-cysteine (via cystathionine as intermediate) into L-methionine by transsulphuration. The CH₃ group of L-methionine originates from the C1 metabolism and is transferred to L-homocysteine by the MetE or MetH methionine synthases (review: Greene RC (1996) in Neidhardt FC et al. (eds) "Escherichia coli and Salmonella", 2nd edition, pp 542-560). Strains and processes for the fermentative production of L-methionine have been described for *E. coli*, for example, in WO2006/001616 or WO2009/043803.

The shikimate metabolic pathway for synthesizing *inter alia* chorismate, enterobactin, menaquinone, tetrahydrofolate, ubiquinone and the aromatic amino acids is likewise tightly regulated. Aromatic biosynthesis in *E. coli* is regulated both at the genetic level by attenuation and repression, and by feeback inhibition of enzymes (Frost and Draths, Annual review of microbiology, 49:557-79 (1995); Pittard, Genes to Cells 1(8):717-25 (1996)). This pathway matches the production of aromatic metabolites exactly to the requirements of the cell. The shikimic acid pathway is regulated above all by controlling the initial reaction which is catalysed by three different isoenzymes of 3-deoxy-D-arabino-heptulosonate-7-phosphate synthase (DAHP synthase, encoded by the aroF, aroG and aroH genes). The AroG gene product constitutes 80% of the total activity of all DAHP synthases in *E. coli* (Tribe et al., Journal of Bacteriology 127(3): 1085-1097 (1976); Pittard, Genes to Cells 1(8):717-25 (1996));

AroG is 95% inhibited by the aromatic amino acid L-phenylalanine. At the genetic level, the TyrR regulator, in the presence of phenylalanine and tryptophan, represses aroG transcription (Baseggio et al., Journal of Bacteriology 172(5):2547-57 (1990); Davies et al., Gene 33(3):323-31 (1985)). The applications EP 1 270 721 and EP 2 147 972 describe the beneficial effect of employing an aroG allele on producing and preparing the aromatic amino acids L-phenylalanine and L-tryptophan using strains of the genus *Escherichia.*

EP 2 204 441 A1 teaches that an increase of the (p)ppGpp level in bacteria cells leads to a decrease of the synthesis of stable RNA and an increase of the expression of some genes relevant for the amino acid synthesis and that the attenuation of the PSII activity, i.e. of the whole gene product of the spoT gene, leads to an improvement of the synthesis of heterologous genes by these.

Gentry and Cashel (D.R. Gentry and M. Cashel, Molecular Microbiology (1996) 19(6), 1373-1384) essentially teach that the spoT gene product contains a distinct region that is responsible for the ppGppase activity and a distinct, but overlapping region that is responsible for the PSII (i.e. (p)ppGpp synthetase) activity of this gene product.

Against this background, the object on which the present invention is based is to provide a cell which is improved over the cells described in the prior art, methods of preparing such a cell and methods using such a cell for overproducing methionine or tryptophan, said improvement relating to factors such as the intracellular concentration attained of the overproduced amino acid, the yield of the overproduced amino acid after processing the culture, the growth properties of the cell and the time and number of method steps required for overproducing and processing said amino acid, and the resources required by the process, for example with regard to time, energy and amount of the strains and reactants used.

This object is achieved by by an E. coli cell which already overproduces methionine or tryptophan,
and wherein the activity of at least one ppGppase from the group comprising- the amino acid sequences- SEQ ID NO:2, SEQ ID NO:4 and SEQ ID NO:6 is reduced as a result of said ppGppase having the following modifications: an insertion of the two amino acids, His and Asn, is present between Asp84 and Met85.

In a further embodiment the problem is solved by an *E. coli* cell, which already overproduces methionine or tryptophan, wherein the activity of the ppGppase according to SEQ ID NO:2 is reduced as a result of said ppGppase having the following modifications: an insertion of the two amino acids His and Asn is present between Asp84 and Met85 and preferably has at least one modification from the group comprising substitutions, preferably non-conservative substitutions, at the amino acids Gln9, Thr13, Tyr63, Arg109, Gln225,
Cys245, Val248, Asn268, Ser270, Met280, His344, Pro436, Asn501, Glen505, His543, Ala546, Ser547, Ile548, His555, Gly556, His557, Pro559, Lys619, Thr621, Ala622, Thr627, Thr651, Ala669, Ala675, Thr698 and an insertion between Glu343 and His344.

The ppGppase of the E. coli cell according to the present invention may further have at least one modification from the group having the following substitutions or substitutions of amino acids homologous to the amino acid to be substituted by the same amino acids:
1.) substitution of Gln9 by an amino acid selected from the group consisting of Leu, Ile and Val, preferably Leu,
2.) substitution of Thr13 by an amino acid selected from the group consisting of Lys, Arg, His, Gin and Asn, preferably Arg or Asn,
3.) substitution of Tyr63 by an amino acid selected from the group consisting of Lys, Arg and His, preferably His,
4.) substitution of Arg109 by an amino acid selected from the group consisting of Gin and Asn, preferably Gin,
5.) substitution of Gln225 by an amino acid selected from the group consisting of Ser, Ala and Thr, preferably Thr,
6.) substitution of Cys245 by an amino acid selected from the group consisting of Leu, Ile and Val, preferably Leu,
7.) substitution of Val248 by an amino acid selected from the group consisting of Lys, Arg and His, preferably His,
8.) substitution of Asn268 by an amino acid selected from the group consisting of Lys, Arg and His, preferably L-His or Lys,
9.) substitution of Ser270 by an amino acid selected from the group consisting of Ala, Leu, Ile and Val, preferably Ala or Val,
10.) substitution of Met280 by an amino acid selected from the group consisting of Ser, Thr and Ala, preferably Ala,
11.) insertion of the amino acids Lys and Glu between amino acids Glu343 and His344,
12.) substitution of His344 by an amino acid selected from the group consisting of Gln and Asn, preferably Gln,
13.) substitution of Pro436 by an amino acid selected from the group consisting of Ser, Ala and Thr, preferably Ser,
14.) substitution of Asn501 by an amino acid selected from the group consisting of Ser, Ala and Thr, preferably L-alanine,
15.) substitution of Gln505 by an amino acid selected from the group consisting of Pro, Ser, Ala and Thr, preferably Pro or Ala,
16.) substitution of His543 by an amino acid selected from the group consisting of Asn and Gin, preferably Gln,
17.) substitution of Ala546 by an amino acid selected from the group consisting of Asn and Gln, preferably Gln,
18.) substitution of Ser547 by an amino acid selected from the group consisting of Ala, Leu, Ile and Val, preferably Ala or Val,
19.) substitution of Ile548 by an amino acid selected from the group consisting of Asn and Gin, preferably Asn,
20.) substitution of His555 by an amino acid selected from the group consisting of Leu, Ile and Val, preferably Val,
21.) substitution of Gly556 by Lys, Arg and His, preferably Arg,
22.) substitution of His557 by an amino acid selected from the group consisting of Asn and Gin, preferably Gln,
23.) substitution of Pro559 by an amino acid selected from the group consisting of Ser, Ala and Thr, preferably Ala,
24.) substitution of Lyes619 by an amino acid selected from the group consisting of Asn and Gln, preferably Gln,
25.) substitution of Thr621 by an amino acid selected from the group consisting of Leu, Ile and Val, preferably Ile,
26.) substitution of Ala622 by an amino acid selected from the group consisting of Glu and Asp, preferably Asp,
27.) substitution of Thr627 by an amino acid selected from the group consisting of Ala and Gly, preferably Ala,
28.) substitution of Thr651 by an amino acid selected from the group consisting of Glu and Asp, preferably Glu,
29.) substitution of Ala669 and/or Ala675 by Thr,
30.) substitution of Thr698 by an amino acid selected from the group consisting of Gin and Asn, preferably Asn.

The *E. coli* cell according to the present invention preferably overexpresses, relative to its wild type, at least one nucleic acid sequence that codes for any of the following enzymes:
1) thiosulphate/sulphate transport system CysPUWA (EC 3.6.3.25),
2) 3'-phosphoadenosine 5'-phosphosulphate reductase CysH (EC 1.8.4.8),
3) sulphite reductase CysJI (EC 1.8.1.2),
4) cysteine synthase A CysK (EC 2.5.1.47),
5) cysteine synthase B CysM (EC 2.5.1.47),
6) serine acetyltransferase CysE (EC 2.3.1.30),
7) glycine cleavage system GcvTHP-Lpd (EC 2.1.2.10, EC 1.4.4.2, EC 1.8.1.4),
8) lipoyl synthase LipA (EC 2.8,1.8),
9) lipoyl-protein ligase LipB (EC 2.3.1.181),
10) phosphoglycerate dehydrogenase SerA (EC 1.1.1.95),
11) 3-phosphoserine phosphatase SerB (EC 3.1.3.3),
12) 3-phosphoserine/phosphohydroxythreonine aminotransferase SerC (EC 2.6.1.52),
13) serine hydroxymethyltransferase GlyA (EC 2.1.2.1),
14) aspartokinase I and homoserine dehydrogenase I ThrA (EC 2.7.2.4, EC 1.1.1.3),
15) aspartate kinase LysC (EC 2.7.2.4),
16) homoserine dehydrogenase Hom (EC 1.1.1.3),
17) homoserine O-acetyltransferase MetX (EC 2.3.1.31),
18) homoserine O-succinyltransferase MetA (EC 2.3.1.46),
19) cystathionine gamma-synthase MetB (EC 2.5.1.48),
20) β C-S lyase AecD (EC 4.4.1.8, also referred to as beta-lyase),
21) cystathionine beta-lyase MetC (EC 4.4.1.8),
22) B12-independent homocysteine *S*-methyltransferase MetE (EC 2.1.1.14),
23) B12-dependent homocysteine S-methyltransferase MetH (EC 2.1.1.13),
24) methylenetetrahydrofolate reductase MetF (EC 1.5.1.20),
25) L-methionine exporter BrnFE from *Corynebacterium glutamicum,*
26) valine exporter YgaZH from *Escherichia coli,* preferably one represented by the database codes b2682, b2683 or variants thereof,
27) putative transporter YjeH from *Escherichia coli,* preferably one represented by the database code b4141 or variants thereof,
28) pyridine nucleotide transhydrogenase PntAB (EC 1.6.1.2),
29) O-succinylhomoserine sulphhydrylase MetZ (EC 2.5.1.48),
30) phospho*enol*pyruvate carboxylase Pyc (EC 4.1.1.31),
31) thiosulphate sulphurtransferase RDL2 (EC 2.8.1.1),
32) thiosulphate-thiol sulphurtransferase (EC 2.8.1.3),
33) thiosulphate-dithiol sulphurtransferase (EC 2.8.1.5).

The E. coli cell according to the present invention preferably expresses on a reduced scale, relative to its wild type, at least one nucleic acid that codes for any of the following enzymes:
1) transcriptional regulator of L-methionine biosynthesis (MetJ), preferably one represented by the database codes b3938, ECK3930 or variants thereof,
2) glucose-6-phosphate isomerase (Pgi, EC 5.3.1.9), preferably one represented by the database codes b4025, ECK4017 or variants thereof,
3) homoserine kinase (ThrB, EC 2.7.1.39), preferably one represented by the database codes b0003, ECK0003 or variants thereof,
4) *S*-adenosylmethionine synthase (MetK, EC 2.5.1.6), preferably one represented by the database codes b2942, ECK2937 or variants thereof,
5) dihydrodipicolinate synthase (DapA, EC 4.2.1.52), preferably one represented by the database codes b2478, ECK2474 or variants thereof,
6) phospho*enol*pyruvate carboxykinase (Pck, EC 4.1.1.49), preferably one represented by the database codes b3403, ECK3390 or variants thereof,
7) formyltetrahydrofolate hydrolase (PurU, EC 3.5.1.10), preferably one represented by the database codes b1232, ECK1227 or variants thereof,
8) pyruvate kinase II (PykA, EC 2.7.1.40), preferably one represented by the database codes b1854, ECK1855 or variants thereof,
9) pyruvate kinase I (PykF, EC 2.7.1.40), preferably one represented by the database codes b1676, ECK1672 or variants thereof,
10) subunit of L-methionine transporter (MetQNI), preferably one represented by the database codes b0197, ECK0197 or variants thereof,
11) subunit of L-methionine transporter (MetQNI), preferably one represented by the database codes b0198, ECK0198 or variants thereof,
12) subunit of L-methionine transporter (MetQNI), preferably one represented by the database codes b0199, ECK0199 or variants thereof,
13) deoxycytidine 5'-triphosphate deaminase (Dcd, EC 3.5.4.13), preferably one represented by the database codes b2065, ECK2059 or variants thereof,
14) putative N-acyltransferase (YncA), preferably one represented by the database codes b1448, ECK1442 or variants thereof,
15) regulatory sRNA FnrS, preferably one represented by the database codes b4699, ECK4511 or variants thereof,
16) sigma factor RpoS, preferably one represented by the database codes b2741, ECK2736 or variants thereof.

The E. coli cell according to the present invention preferably overexpresses, relative to its wild type, at least one nucleic acid sequence of the following enzymes:
1) anthranilate synthase (trpDE, EC 4.1.3.27), anthranilate phosphoribasyl-transferase (trpD, EC 2.4.2.18), phosphoribosylanthranilate isomerase (trpC, EC 5.3.1.24), indole-3-glycerol-phosphate synthase (trpC, EC 4.1.1.48) and tryptophan synthase (trpAB, EC 4.1.2.8 and 4.2.1.122),
2) phosphoglycerate dehydrogenase SerA (EC 1.1.1.95),
3) 3-phosphoserine phosphatase SerB (EC 3.1.3.3),
4) 3-phosphoserine/phosphohydroxythreonine aminotransferase SerC (EC 2.6.1.52),
5) L-tyrosine-sensitive DHAP synthase (aroF, EC 2.5.1.54),
6) L-phenylalanine feedback-resistant DHAP synthase (aroG, EC 2.5.1.54),
7) L-tryptophan-sensitive DHAP synthase (aroH, EC 2.5.1.54),
8) phospho*enol*pyruvate synthase ppsA (EC 2.7.9.2),
9) phosphoenolpyruvate carboxykinase pck (EC 4.1.1.49),
10) transketalase A tktA (EC 2.2.1.1),
11) transketolase B tktB (EC 2.2.1.1),
12) gene product of the *E. coli* open reading frame (ORF) yddG, preferably one represented by the database codes b1473, ECK1467 or variants thereof.

The E. coli cell according to the present invention preferably expresses on a reduced scale, relative to its wild type, at least one nucleic acid sequence that codes for any of the following enzymes:
1) tryptophanase (tnaA, EC 4.1.99.1),
2) repressor of the trp operon (trpR), preferably one represented by the database codes b4393, ECK4385 or variants thereof,
3) chorismate mutase T or prephenate dehydrogenase (tyrA, EC 1.3.1.12),
4) chorismate mutase P or prephenate dehydrogenase (pheA, EC 4.2.1.51),
5) tryptophan-specific transport protein mtr, preferably one represented by the database codes b3161, ECK3149 or variants thereof,
6) tryptophan permease (tnaB), preferably one represented by the database codes b3709, ECK3702 or variants thereof,
7) transporter for aromatic amino acids (aroP), preferably one represented by the database codes b0112, ECK0111 or variants thereof,
8) L-serine deaminase (sdaA, EC 4.3.1.17),
9) glucose-6-phosphate isomerase (pgi, EC 5.3.1.9),
10) tyrosine aminotransferase (thrB), preferably one represented by the database codes b4054, ECK4046 or variants thereof,
11) repressor of the glp regulon (glpR), preferably one represented by the database codes b3423, ECK3409 or variants thereof,
12) sigma factor RpoS (rpoS), preferably one represented by the database codes b2741, ECK2736 or variants thereof.

The problem addressed by the present invention is further solved by a method of preparing methionine or tryptophan, comprising the step of culturing an *E. coli* cell which already overproduces methionine, or tryptophan, and is genetically modified over its wild type in such a way that it has a reduced ppGppase activity relative to its wild type and wherein the *E. coli* cell has a (p)ppGpp- synthetase activity wich is essentially unchanged relative to its wild type, wherein said ppGppase is preferably selected from the group comprising the amino acid sequences SEQ ID NO:2, SEQ ID NO:4 and SEQ ID NO:6 is reduced as a result of said ppGppase having at least the following modification: an insertion of the two amino acids His and Asn is present between Asp84 and Met85 with optional purification of the amino acid.

The inventors of the present invention have found that the production output of a cell overproducing methionine or tryptophan, is surprisingly increased by a situation in which said cell has a reduced ppGppase activity relative to its wild type. Surprisingly, this is the case particularly if the (p)ppGpp-synthetase activity of the cell is essentially unchanged relative to its wild type.

An essential property of the cell according to the invention is that of being genetically modified over its wild type in such a way that it has a reduced ppGppase activity relative to its wild type. In a preferred embodiment, the term "ppGppase activity", as used herein, means the enzymatic activity of a guanosine-3',5'-bis(diphosphate) 3'-diphosphatase (EC 3.1.7.2), *i.*e. the ability to catalyse the breakdown of guanosine-3',5'-bis(diphosphate) (=ppGpp) to give GDP and pyrophosphate. In a preferred embodiment, the term "ppGppase", as used herein, means an enzyme having ppGppase activity, which enzyme may have ppGppase activity only or a plurality of activities, including ppGppase activity. ppGpp is produced with the action of a guanosine-5'-triphosphate, 3'-diphosphate pyrophosphatase (EC 3.6.1.40) from guanosine 3'-diphosphate 5'-triphosphate (=pppGpp), a compound which in turn is produced by GTP diphosphokinases (EC 2.7.6.5, frequently abbreviated to PSI and PSII in the literature) from ATP and GTP. In a preferred embodiment, the term "(p)ppGpp-synthetase activity" comprises the ability, as used herein, to produce at least one, preferably both, of the two compounds pppGpp and ppGpp, preferably with hydrolysis of ATP. The prior art describes numerous ppGppases and genes coding for ppGppases, for example in Blattner et al. (Science 277: 1453-1462 (1997)) and in databases, for example NC_003197 (REGION: 3934070-3936181), NC_009832 (REGION: (5391421-5393532), NC_007613? EC_004741, NC_013971 and NC_009648. In a preferred embodiment, the wording that a cell which is genetically modified over its wild type in such a way that it has a reduced ppGppase activity relative to its wild type and "overproduces methionine or tryptophan", as used herein, means that the starting strain on which the cell is based already produces an increased amount of the relevant amino acid compared to its original wild-type strain, even before and independently of the reduction in ppGppase activity.

A person skilled in the art may determine the activity of an enzyme, for example of a ppGppase or a (p)ppGpp synthetase, on the basis of activity assays, like those which have been described in conjunction with methods of expert evaluation and interpretation of the kinetic parameters obtained with such determinations in the prior art, for example Cornish-Bowden, Fundamentals of Enzym Kinetics, Portland Press Limited, 1995. The prior art furthermore describes specific assays for determining the activity of (p)ppGpp synthetases (Mechold et al., 1996, J. Bact. 178, 1401-1411) and ppGppase (Gallant et al., 1970, Cold Spring Harbor Symp. Quant. Biol. 35, 397). Briefly, this involves labelling the cells having the activity to be determined with radiolabelled nucleotides, starving them with regard to isoleucine by adding 400 µm/ml valine, followed by extracting the cell's nucleotide pool after 15 minutes of valine inhibition in 0.66 N formic acid and subsequent fractionation of pppGpp, ppGpp, pppG and pppA by means of thin-layer chromatography on PEI-cellulose plates in 1.5 M potassium dihydrogen phosphate and visualization using autoradiography. In a preferred embodiment, "a cell which is genetically modified over its wild type in such a way that it has a reduced ppGppase activity relative to its wild type" means that such a cell under comparable conditions has less ppGppase activity, measured by way of the number of ppGpp molecules hydrolysed per unit time, than a wild-type cell, that is, in the order of increasing preference, less than 70, 60, 40, 20, 10 or 5% of the activity of a wild-type cell. In a further preferred embodiment, "a cell which has a (p)ppGpp-synthetase activity which is essentially unchanged relative to its wild type" means that said cell under comparable conditions has at least 90, 95, 99, 101, 105, 110% of the activity of a wild-type cell, measured by way of the number of (p)ppGpp molecules synthesized per unit time. In a particularly preferred embodiment, the cell is an *E. coli* strain, whose ppGppase activity of the sequence SEQ ID NO. 2 or a variant is reduced, while the activity of the (p)ppGpp synthetase of said cell is essentially unchanged.

The introduction of specific mutations into biological macromolecules, at both the nucleic acid and amino acid levels, is nowadays part of the routinely used standard methods of molecular biology, microbiology and biochemistry. For example, methods of site-directed mutagenesis using mutagenic oligonucleotides (T. A. Brown: Gentechnologie für Einsteiger [Genetic engineering for beginners], Spektrum Akademischer Verlag, Heidelberg, Germany, 1993) or polymerase chain reaction (PCR), as described in the manual by Gait: Oligonucleotide synthesis: A Practical Approach (IRL Press, Oxford, UK, 1984) or by Newton and Graham (PCR, Spektrum Akademischer Verlag, Heidelberg, 1994) may be used. To engineer mutations, the Quick Change Site-Directed Mutagenesis kit from Stratagene (Amsterdam, Netherlands) may be used, for example. Using these methods involves amplifying the starting sequence, for example a ppGppase-encoding sequence described in the prior art, with the aid of the polymerase chain reaction (PCR) starting from isolated total DNA of a wild-type strain, cloning said sequence into suitable plasmid vectors, and then subjecting the DNA to the mutagenesis process. By means of "GeneSOEing" (Gene Splicing by Overlap Extension, Horton, Molecular Biotechnology 3: 93-98 (1995)), the point mutations may even be obtained by PCR. A de *novo* gene synthesis (for example by GENEART AG, Regensburg, Germany) of the nucleotide sequences may also be used for producing mutations in the spoT gene. The mutations generated can be determined and checked by DNA sequencing, for example by the method of Sanger et al. (Proc. Natl. Acad. Sci. 74 (12): 5463-5467, 1977). The alleles generated may be incorporated into the chromosome of appropriate strains, for example by transformation and the method of gene or allele replacement.

A customary method, described by Hamilton et al. (Journal of Bacteriology 171, 4617 - 4622 (1989)), is the method of gene replacement with the aid of a conditionally replicating pSC101 derivative pMAK705 or with pKO3 (Link et al., Journal of Bacteriology 179: 6228-6237). Other methods described in the prior art, for example that of Martinez-Morales et al. (Journal of Bacteriology 1999, 7143-7148 (1999)) or that of Boyd et al. (Journal of Bacteriology 182, 842-847 (2000)), may likewise be utilized.

Another customary method consists of incorporating via short, homologous flanking sequences a DNA fragment generated by PCR or gene synthesis directly into the chromosome with the aid of Lambda Red recombinase, or carrying out a replacement (Proc. Natl. Acad. Sci. 97(12), 6640-6645 (2000); Nature Genetics 20, 123 - 128, 1998).

It is likewise possible to transfer, by conjugation or transduction, the alleles generated into various strains.

Although it is technically feasible to introduce into biological macromolecules numerous artificial nucleotides or amino acids that do not naturally occur in nucleic acid or amino acid sequences, for example in the course of a complete chemical synthesis or by altering the cellular translation apparatus, the present invention, in a preferred embodiment, only provides for proteinogenic L-amino acids for substitutions to replace one or more than one amino acid originally present in the particular polypeptide. In a preferred embodiment, the term "proteinogenic" amino acid means any amino acid out of all the L forms of the amino acids alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine. In a preferred embodiment, the term "homologous" amino acid of an amino acid in the wild-type sequence of a polypeptide, as used herein, means an amino acid which is identified in a different amino acid sequence, which is related to the wild-type sequence with regard to the primary structure however, on the basis of an alignment of the two sequences as being the amino acid corresponding to the amino acid in the wild-type sequence or occupying its corresponding position in the related amino acid sequence. Methods of performing an alignment of two or more amino acid sequences have been described in the prior art, for example in Arthur Lesk (2008), Introduction to bioinformatics, 3rd edition, and comprise the use of software packages such as Clustal W (Thompson et al., Nucleic Acids Research 22, 4637-4680, 1994) or MAFFT (Katoh et al., Genome Information, 16(1), 22-33, 2005). For example, Arg26 in the polypeptide encoded by the human Pax6 gene is homologous to Arg59 of the *Drosophila* gene eyless, *cf.* Lesk (2008), p. 36.

The genetic methods of preparing the mutants according to the invention comprise in addition to genetic engineering methods also traditional genetic methods such as *in vivo* mutagenesis methods using cell populations of *Enterobacteriaceae* strains and mutagens such as N-methyl-N'-nitro-N-nitrosoguanidine (MNNG), ethyl methanesulfonate (EMS), 5-bromouracil or ultraviolet light, for example. Mutagenesis methods are described, for example, in Manual of Methods for General Bacteriology (Gerhard et al. (Eds.), American Society for Microbiology, Washington, DC, USA, 1981) or in Tosaka et al. (Agricultural and Biological Chemistry 42(4), 745-752 (1978)) or in Konicek et al. (Folia Microbiologica 33, 337-343 (1988)). Typical mutagenesis reactions using MNNG comprise concentrations of from 50 to 500 mg/l or else higher concentrations of up to no more than 1 g/l, and an incubation period from 1 to 30 minutes at pH 5.5 to 7.5. Under these conditions, the number of viable cells is reduced by a proportion of approx. 50% to 90% or approx. 50% to 99% or approx. 50% to 99.9% or more.

Mutants or cells are removed from the mutagenized cell population and are propagated. In a further step, their ability to secrete amino acids, preferably methionine or tryptophan, in a batch culture using a suitable nutrient medium is preferably investigated. In the case of uses of suitable robotic systems as described for example in Zimmermann et al. (VDI Berichte No. 1841, VDI-Verlag, Dusseldorf, Germany 2004, 439-443) or Zimmermann (Chemie Ingenenieur Technik 77 (4), 426-428 (2005)), numerous mutants can be investigated in a short time. In general, no more than 3000, no more than 10 000, no more than 30 000 or else no more than 60 000 mutants, where appropriate also more, are investigated. Mutants which, compared with the parent strain or unmutagenized starting strain, exhibit increased secretion of amino acids into the nutrient medium or into the interior of the cell are identified in this way. These include for example mutants whose amino acid secretion is increased by at least 0.5%.

DNA is then prepared or isolated from the mutants, and the corresponding polynucleotide is synthesized with the aid of the polymerase chain reaction using primer pairs which permit amplification of the spoT gene or of the spoT allele according to the invention or of the mutations according to the invention. The DNA is preferably isolated from mutants which secrete amino acids to an increased extent.

In a preferred embodiment, the term "gene", as used herein, means a section on the deoxyribonucleic acid (DNA), which contains the information on production (transcription) of firstly a ribonucleic acid (RNA), with the latter containing the information on production (translation) of a protein (polypeptide), in this case a polypeptide having the activity of a (p)ppGpp synthetase II. The fact that a gene or a polynucleotide contains the information on producing a protein is also referred to as coding of a protein or polypeptide by the gene or by the RNA. Gene expression denotes the biosynthesis of RNA and proteins from the genetic information contained in DNA and genes. Endogenous genes or polynucleotides are understood as meaning the open reading frames (ORFs), genes or alleles or their polynucleotides present in the population of a species. The terms "gene" and "ORF" (open reading frame) are used synonymously in the present invention.

In a preferred embodiment, the term "polynucleotide", as used herein, means a polyribonucleotide and polydeoxyribonucleotide, which may be unmodified RNA or DNA or modified RNA or DNA, with the term polynucleotide being used synonymously and interchangeably with the term "nucleic acid".

In a preferred embodiment, the term "polypeptide", as used herein, means peptides or proteins that include two or more amino acids connected via peptide bonds. The terms polypeptide and protein are used synonymously. Proteins are one of the basic building blocks of all cells. They not only give structure to the cell but are also the molecular "machines" that transport substances, catalyse chemical reactions and recognize signalling agents.

preferably the L form of the amino acid. In a preferred embodiment, the cell according to the invention or the method according to the invention is employed for preparing an essential and/or serine-derived and/or aromatic amino acid. In a preferred embodiment, the term "essential" amino acid means an amino acid which cannot be produced independently by a higher eukaryote, preferably a warm-blooded higher eukaryote, even more preferably a bird or mammal. In a more preferred embodiment, this also includes amino acids which the organism can produce itself, but only if it takes in a suitable precursor, most preferably another amino acid, with the food. In a preferred embodiment, the term "an amino acid derived from serine", as used herein, means an amino acid which is produced via a biosynthetic pathway comprising at least one reaction which consumes serine as reactant, said biosynthetic pathway preferably including only those reactions which directly contribute to the task of the structure of the amino acid to be produced, but not reactions that merely regenerate cofactors.

The cell according to the invention or the cell produced or employed in a method according to the invention may be a cell of numerous organisms which can be employed in biotechnology. In a preferred embodiment, it is a prokaryotic or lower eukaryotic cell. In a preferred embodiment, the term "lower eukaryotic cell", as used herein, means a unicellular eukaryotic cell, preferably a yeast cell. Examples of lower eukaryotic cells include yeasts of the genera *Saccharomyces, Schizosaccharomyces, Candida*, *Picchia* and *Yarrowia.* In another preferred embodiment, the cell is a prokaryotic cell, more preferably a Gram-negative bacterium, even more preferably a cell selected from the group of genera comprising *Escherichia, Erwinia, Providencia* and *Serratia*, even more preferably a cell from the genus *Escherichia*, and most preferably *E. coli.* In a preferred embodiment, it is an isolated cell maintained by way of a pure culture. A further embodiment makes use of a mixture of cultures. In a most preferred embodiment, the cell is a bacterium of the strain EMC1_spoT1849, see Example 4.

The cell according to the invention for preparing at least one amino acid is preferably intact, with unimpaired metabolic activity. However, the teaching according to the invention can also be implemented by using partially lysed and partially purified cells whose metabolism is still capable of producing the amino acid(s) of interest. If the Particular preference is given according to the invention to those E. *coli* cells which produce methionine or tryptophan in an increased amount of the particular amino acid over its wild type taken from nature even before the genetic modification causing a reduced ppGppase activity, *i.*e. in the form of the corresponding starting strain. In a preferred embodiment, the term "starting strain", used interchangeably with the term "parent strain", as used herein, means the microorganism strain on which measures of increasing the productivity of one or more amino acids, peptides or proteins, or measures of increasing the activity of one or more enzymes, for example a measure resulting in overexpression, are carried out. A starting strain may be a wild-type strain, but also a previously modified strain, for example a strain overproducing at least one L-amino acid, which may be used as producer strain.

The prior art has disclosed numerous strains overproducing relevant amino acids and also measures and modifications by which such overproduction can be achieved. In a preferred embodiment, the *E. coli* strain secreting or producing L-methionine has preferably enhanced aspartate kinase (EC 2.7.2.4) enzyme activity, with feedback-resistant alleles being preferred. In *E. coli* there are three different aspartate kinases, encoded by the genes *thrA, metL* and *lysC.* In another preferred embodiment, L-methionine biosynthesis is increased due to attenuation or deletion of the regulatory protein MetJ which is encoded by the *metJ* gene. MetJ is the main repressor of L-methionine biosynthesis in *E. coli.*

In a further preferred embodiment, the cell's starting strain is derived from a strain from the group comprising *Escherichia coli* MG1655, *Escherichia coli* W3110, *Escherichia coli* DH5α, *Escherichia coli* DH10B, *Escherichia coli* BW2952, *Escherichia coli* REL606.

All of the accession numbers and accession codes used for citing nucleic acid and amino acid sequences from the prior art in the present application are accession numbers and accession codes from the BioCyc database of ISR, CA, USA in the version available on-line on 1 December 2011.

In a further preferred embodiment, the cell is derived from the methionine-overproducing *E. coli* producer strain MG1655 ΔmetJ metA*11 Ptrc-metH Ptrc-metF PtrcF-cysPUWAM PtrcF-cysJIH ΔpykF ΔpykA Ptrc09-gcvTHP ΔpurU Ptrc36-ARNmst17-metF comprising the production plasmids pME101-thrA*1-cysE-Pgap-metA*11 and pCC1 BAC-serB-glyA-serA-serC (WO2009/043803).

In a further preferred embodiment, the cell is derived from the methionine-overproducing *E. coli* producer strain MG1655 ΔmetJ Ptrc-metH Ptrc-metF PtrcF-cysPUWAM PtrcF-cysJIH Ptrc09-gcvTHP comprising the production plasmid pME101-thrA*1-cysE-Pgap-metA*11. This strain can be cloned by a number of P1 transductions and curings, as described in the application WO2009/043803. The strain is based on the wild-type strain *E. coli* K12 MG1655. The following modifications were introduced into the genome of this strain: deletion of the gene for the repressor of the L-methionine biosynthesis, *metJ;* insertion of the strong trc promoter upstream of the *metH* gene coding for the cobalamin-dependent methionine synthase; insertion of the strong trc promoter upstream of the *metF* gene coding for 5,10-methylenetetrahydrofolate reductase; insertion of the strong trc promoter upstream of the *cysPUWAM* operon, with *cysPUWA* coding for a sulphate/thiosulphate-uptake transporter and *cysM* coding for cysteine synthase B; insertion of the strong trc promoter upstream of the *cysJIH* operon, with *cysJI* coding for sulphite reductase and *cysH* coding for 3'-phospho-adenylyl-sulphate reductase; insertion of the strong trc09 promoter upstream of the *gcvTHP* operon, with *govT, gcvH* and *gcv P* coding for three components of the glycine-cleavage system.

Cloning of the *E. coli* production plasmid pME101-thrA*1-cysE-Pgap-metA*11 is described in the applications WO2007/077041 and WO2009/043803. It is a low copy plasmid based on the vector pCL1920 (Lerner, C.G. and Inouye, M., Nucl. Acids Res. (1990) 18:4631 [PMID: 2201955]). The empty plasmid, pME101, harbours the *lacl^{q}* gene which codes for a strongly expressed allele of the lac repressor. The *thrA*1* gene was cloned downstream of a strong trc promoter which can be repressed by the Lac repressor. It codes for a feedback-resistant variant of the *E. coli* ThrA aspartate kinase / homoserine dehydrogenase. The *cysE* gene including its natural promoter is located downstream thereof, in the same orientation. It codes for the *E. coli* serine acetyltransferase. Following downstream of *cysE* is the strong *E. coli gapA* promoter which controls expression of the *metA*11* gene. *metA*11* codes for a feedback-resistant variant of the *E. coli* homoserine O-succinyltransferase.

Examples of other L-methionine-secreting and producing microorganisms which may be mentioned are the following strains: *E. coli* TF4076BJF metA#10 + metYX(Lm) (WO2008/127240); *E. coli* W3110ΔJ/pKP451 (EP 1 445 310 B1, page 7, example 4); *E. coli* WΔthrBCΔmetJmetK32 pMWPthrmetA4Δ5Δ9 (Yoshihiro Usuda and Osamu Kurahashi, 2005, Applied and Environmental Microbiology, Vol. 71, No. 6, p. 3228-3234); and W3110/pHC34 (WO01/27307 page 13, example 3). Further examples of various suitable microorganisms are described in Gomes et al. (Enzyme and Microbial Technology 37, 2005, 3-18).

The prior art also describes tryptophan-overproducing strains and also measures and modifications by which overproduction of this amino acid can be achieved, for example *E. coli* JP4735/pMU3028 (US5,756,345), *E. coli* JP6015/pMU91 (US5,756,345), *E. coli* SV164(pGH5) (WO94/08031), *E. coli* AGX17(pGX44) (NRRL B-12263) (US4,371,614), *E. coli* AGX6(pGX50)aroP (NRRL B-12264) (US4,371,614), *E. coli* AGX17/pGX50,pACKG4-pps (WO97/08333), *E. coli* ATCC 31743 (CA1182409), *E. coli* C534/pD2310,pDM136 (ATCC 39795) (WO87/01130), *E. coli* JB102/p5LRPS2 (US5,939,295).

In a preferred embodiment, L-tryptophan-overproducing strains of the *Enterobacteriaceae* family have one or more than one genetic phenotypical feature selected from the group comprising resistance to 5-methyl-DL-tryptophan, resistance to 5-fluorotryptophan, resistance to 4-methyl-DL-tryptophan, resistance to 6-methyl-DL-tryptophan, resistance to 4-fluorotryptophan, resistance to 6-fluorotryptophan, resistance to anthranilate, resistance to tryptazan, resistance to indole, resistance to indoleacrylic acid, need for phenylalanine, need for tyrosine, optionally ability to utilize sucrose, enhancement of the tryptophan operon, preferably of anthranilate synthase, preferably of the feedback-resistant form, a partly defective tryptophanyl-tRNA synthase, an attenuated tryptophan uptake, a defective tryptophanase, inactivated repressor proteins, enhancement of serine biosynthesis, enhancement of phospho*eno*/pyruvate synthesis, enhancement of D-erythrose-4-phosphate synthesis, enhancement of 3-deoxy-D-arabino-heptulosonate-7-phosphate (DHAP) synthesis, and enhancement of chorismate biosynthesis.

Besides at least one genetic modification causing a reduction in ppGppase activity, the cell according to the invention may have further modifications over the starting strain which effect an increase in production of the amino acid(s) of interest, in particular by altering gene expression. In a preferred embodiment, the wording that the cell is genetically modified over its wild type in such a way that it "expresses to a reduced degree or overexpresses at least one nucleic acid sequence or a variant thereof", relative to its wild type, as used herein, means that the genetically modified cell produces a lower or a higher amount of transcription and/or translation product of the nucleic acid sequence than the genetically unmodified cell would do, under comparable conditions, for example temperature, supply of nutrients and cell density.

Expression of a nucleic acid can be increased, for example, by increasing the copy number of the corresponding polynucleotides chromosomally or extrachromosomally by at least one copy. A widely used method of increasing the copy number consists of inserting the relevant polynucleotide into a vector, preferably a plasmid, which is replicated by a bacterium. Examples of plasmid vectors suitable for *Enterobacteriaceae* are pACYC184-derived cloning vectors (Bartolomé et al.; Gene 102: 75-78 (1991)), pTrc99A (Amann et al.; Gene 69: 301-315, 1988) or pSC101 derivatives (Vocke and Bastia; Proc. Natl. Acad. Sci. 80(21): 6557-6561, 1983). Plasmids derived from pCL1920 (Lerner, C.G. and Inouye, M., Nucl. Acids Res., 1990, 18:4631 [PMID: 2201955]) are also particularly suitable. Plasmid vectors derived from "bacterial artificial chromosomes" (BACs), for example pCC1BAC (EPICENTRE Biotechnologies, Madison, U.S.A.), are likewise suited to increasing the copy number of the relevant polynucleotides in *E. coli.*

Vectors which may furthermore be employed are transposons, insertion elements (IS elements) or phages. These types of genetic systems are illustrated, for example, in the patents US 4,822,738, US 5,804,414 and US 5,804414. Similarly, the IS element ISaBI described in WO 92/02627 or the Tn 45 transposon of plasmid pXZ10142. (cited in "Handbook of *Corynebacterium glutamicum"* (editors: L. Eggeling and M. Bott)) may be used.

Another common method of increasing expression is the method of chromosomal gene amplification. This method involves inserting at least one additional copy of the polynucleotide of interest into the chromosome of a cell. Amplification processes of this type are described in WO 03/014330 or WO 03/040373, for example.

Another method of increasing expression consists of functionally linking the relevant gene or allele to a promoter or an expression cassette (operably linked). Promoters suitable for *E. coli* are, for example, the promoters described by Amann et al. (Gene 69(2), 301-315, 1988) and Amann and Brosius (Gene 40(2-3), 183-190, 1985), T3, T7, SP6, M13, lac, tac and trc. A promoter of this type may be inserted, for example, upstream of the gene in question, typically at a distance of approximately 1 - 500 nucleotide bases from the start codon. US 5,939,307 reports that incorporation of expression cassettes or promoters such as, for example, tac promoter, trp promoter, Ipp promoter or PL promoter and PR promoter of λ phage, for example upstream of the chromosomal threonine operon, achieved an increase in expression. The T7-phage promoters, the gear-box promoters, the nar promoter or the promoters of the genes rrsG, rnpB, csrA, csrB, ompA, fusA, pepQ, rplX and rpsG may be used in a similar way. These types of expression cassettes or promoters may also be used in order to overexpress plasmid-bound genes, as described in EP 0 593 792. By using the laclQ allele, expression of genes can in turn be controlled (Glascock and Weickert, Gene 223, 221-231, 1998). A person skilled in the art may furthermore increase the activity of said promoters by modifying their sequence by means of one or more nucleotide substitutions, by insertion(s) and/or deletion(s).

The degree of expression of a nucleic acid or a variant thereof can be reduced by suitable culture management, by genetic modification (mutation) of the signal structures of gene expression or else by antisense-RNA technology. Signal structures of gene expression are, for example, repressor genes, activator genes, operators, promoters, attenuators, ribosomal binding sites, the start codon and terminators. The person skilled in the art finds information on these, *inter alia*, for example in Jensen and Hammer (Biotechnology and Bioengineering 58: 191-195, 1998), in Carrier and Keasling (Biotechnology Progress 15: 58-64, 1999), Franch and Gerdes (Current Opinion in Microbiology 3: 159-164, 2000), Kawano et al. (Nucleic Acids Research 33(19), 6268-6276, 2005) and in known textbooks of genetics and molecular biology such as, for example, the textbook of Knippers ("Molekulare Genetik" [Molecular Genetics], 6th edition, Georg Thieme Verlag, Stuttgart, Germany, 1995) or that of Winnacker ("Gene und Klone" [Genes and Clones], VCH Verlagsgesellschaft, Weinheim, Germany, 1990). In a preferred embodiment, the term "knock out" of a gene, as used herein, means that the gene or the cellular machinery required for its expression or required nucleic acids or parts thereof, for example promoters, have been genetically modified, for example by mutation, deletion or insertion, in such a way that expression is reduced, in the order of increasing preference, by 50, 75, 80, 85, 90, 95 or 99%. It is possible, for example, for expression of the gene to be put under the control of a promoter which leads to reduced expression in the microorganism used for the method in comparison with the starting strain. Expression may furthermore be reduced by deleting the gene or parts thereof in the microorganism used for the method. It is also possible to use transitions, transversions or insertions that lead to missense mutations or nonsense mutations. Expression may furthermore be reduced by using a ribosome-binding site which leads to reduced translation in the microorganism used for the method in comparison with the starting strain. A person skilled in the art may furthermore reduce expression of the gene by worsening the codon usage in respect of the microorganism used for the method. Finally, gene expression may be, is reduced by suppressing a stop-codon mutation in the coding region of the gene by means of suitable t-RNA suppressors.

The cell according to the invention or used according to the invention exhibits increased secretion or production of the desired amino acid in a fermentation process in comparison with the starting strain or parent strain employed, with the amino acids being released into the surrounding medium or accumulated inside the cell.

The performance of the cell according to the invention or of the fermentation process using the same with respect to one or more of the parameters selected from the group consisting of product concentration (product per volume), product yield (product formed per carbon source consumed) and product formation (product formed per volume and time) or else other process parameters and combinations thereof is improved by at least 0.5%, at least 1%, at least 1.5% or at least 2% based on the starting strain or parent strain or the fermentation process using the same.

The cell may be cultured in the method according to the invention continuously - as described, for example, in PCT/EP2004/008882 - or discontinuously in a batch process (batch cultivation) or a fed-batch process or a repeated fed-batch process, for the purpose of producing L-amino acids. A general summary of known culturing methods is available in the textbook by Chmiel (Bioprozesstechnik 1. Einführung in die Bioverfahrenstechnik [Bioprocess Technology 1. Introduction to Bioprocess Technology] (Gustav Fischer Verlag, Stuttgart, 1991)) or in the textbook by Storhas (Bioreaktoren und periphere Einrichtungen [Bioreactors and Peripheral Equipment] (Vieweg Verlag, Brunswick/Wiesbaden, 1994)).

The culture medium or fermentation medium to be used must suitably satisfy the requirements of the particular strains. Descriptions of media for culturing different microorganisms are given, for example, in the manual "Manual of Methods for General Bacteriology" published by the American Society for Bacteriology (Washington D.C., USA, 1981). The terms culture medium, fermentation medium and medium are mutually interchangeable.

The carbon source employed may be sugars and carbohydrates, for example glucose, sucrose, lactose, fructose, maltose, molasses, sucrose-containing solutions derived from sugar beet or sugar cane production, starch, starch hydrolysate and cellulose, oils and fats, for example soybean oil, sunflower oil, peanut oil and coconut oil, fatty acids, for example palmitic acid, stearic acid and linoleic acid, alcohols, for example glycerol, methanol and ethanol, and organic acids, for example acetic acid. These substances may be used individually or as mixtures.

The nitrogen source employed may be organic nitrogen-containing compounds, such as peptones, yeast extract, meat extract, malt extract, cornsteep liquor, soybean flour and urea, or inorganic compounds, such as ammonium sulphate, ammonium chloride, ammonium phosphate, ammonium carbonate, ammonium nitrate, ammonia and ammonium thiosulphate. The nitrogen sources may be used individually or as mixtures.

The phosphorus source employed may be phosphoric acid, potassium dihydrogen phosphate or dipotassium hydrogen phosphate or the corresponding sodium-containing salts.

The sulphur source employed may be a salt of dithiosulphuric acid (thiosulphate), either alone or together with other sulphur sources, for example sulphate, sulphite, sulphide, hydrogen sulphide, methanethiolate or dithionite, see also EP application 11151526.8.

The culture medium must furthermore contain salts, for example in the form of chlorides, of metals, for example sodium, potassium, magnesium, calcium and iron, for example magnesium sulphate or iron sulphate, which are necessary for growth. Finally, growth substances such as amino acids, for example homoserine, and vitamins, for example cobalamin, thiamine, biotin or pantothenic acid, may be used in addition to the substances mentioned above.

In addition to this, suitable precursors of the particular amino acid may be added to the culture medium.

The employed substances mentioned may be added to the culture in the form of a once-only mixture or fed in in a suitable manner during culturing.

Basic compounds such as sodium hydroxide, potassium hydroxide, ammonia or aqueous ammonia, or acidic compounds such as phosphoric acid or sulphuric acid, are employed in a suitable manner for controlling the pH of the culture. In general, the pH is adjusted to a value of from 6.0 to 9.0, preferably of from 6.5 to 8. It is possible to use antifoams, such as fatty acid polyglycol esters, for controlling foam formation. Suitable substances which act selectively, for example antibiotics, may be added to the medium in order to maintain the stability of plasmids. In order to maintain aerobic conditions, oxygen or oxygen-containing gas mixtures, such as air, are passed into the culture. It is also possible to use liquids which are enriched with hydrogen peroxide. Where appropriate, the fermentation is conducted under positive pressure, for example under a pressure of 0.03 to 0.2 MPa. The temperature of the culture is normally from 20°C to 45°C, and preferably from 25°C to 40°C. In the case of batch processes, the culture is continued until a maximum of the desired amino acid has been produced. This objective is normally achieved within from 10 hours to 160 hours. Longer culturing times are possible in the case of continuous processes.

Suitable fermentation media are described, *inter alia,* in US 6,221,636, US 5,840,551, US 5,770,409, US 5,605,818, US 5,275,940 and US 4,224,409.

The fermentation broth prepared in this way is then subjected to further processing into a solid or liquid product.

In a preferred embodiment, a "fermentation broth", as used herein, is understood as meaning a fermentation medium in which a microorganism has been cultured for a certain time and at a certain temperature. The fermentation medium, or the medium employed during the fermentation, contains all the substances or components which are required for propagation of the microorganism and formation of the desired amino acid.

At the conclusion of the fermentation, the resulting fermentation broth accordingly contains a) the biomass of the microorganism which has been produced due to propagation of the cells of said microorganism, b) the desired amino acid formed during the fermentation, c) the by-products formed during the fermentation, and d) the constituents of the fermentation medium/fermentation media employed, or of the employed substances, for example vitamins, such as biotin, amino acids such as homoserine, or salts such as magnesium sulphate, which constituents were not consumed by the fermentation.

The by-products include substances which are generated by the microorganisms employed in the fermentation in addition to the particular desired L-amino acid, and which may be secreted. They include L-amino acids which amount to less than 30%, 20% or 10% in comparison with the desired amino acid, furthermore organic acids having from one to three carboxyl groups, for example acetic acid, lactic acid, citric acid, malic acid or fumaric acid, and also sugars, for example trehalose.

Typical fermentation broths which are suitable for industrial purposes have an amino acid content of from 40 g/kg to 180 g/kg or from 50 g/kg to 150 g/kg. In general, the biomass content (by way of dry biomass) is from 20 to 50 g/kg.

The prior art has described numerous processes for purifying the amino acid from the culture broth or fermentation broth produced, which include ion exchange chromatography, crystallization, extraction processes and treatment with activated carbon. This results in largely pure L-amino acids having a content of ≥ 90% by weight, ≥ 95% by weight, ≥ 96% by weight, ≥ 97% by weight, ≥ 98% by weight or ≥ 99% by weight.

It is likewise possible to purify an amino acid from the fermentation broth produced by removing the biomass of the bacterium contained in the fermentation broth completely (100%) or almost completely, *i*.*e*. more than or greater than (>) 90%, > 95%, > 97%, > 99%, and leaving the other constituents of the fermentation broth largely, *i.*e. 30% - 100%, 40% - 100%, 50% - 100%, 60% - 100%, 70% - 100%, 80% - 100%, or 90% - 100% thereof, preferably greater than or equal to (≥) 50%, ≥ 60%, ≥ 70%, ≥ 80%, ≥ 90% or ≥ 95%, or else completely (100%) in the product.

Separation methods such as, for example, centrifugation, filtration, decanting, flocculation or a combination thereof are employed to remove or separate off the biomass.

The broth obtained is then thickened or concentrated using known methods such as, for example, with the aid of a rotary evaporator, thin-layer evaporator, falling film evaporator, by reverse osmosis, by nanofiltration or a combination thereof.

This concentrated broth is then worked up by methods of freeze-drying, spray drying, spray granulation or by other processes to give a preferably pourable, finely divided powder. This pourable, finely divided powder can then in turn be converted into a coarse-grain, readily pourable, storable and largely dust-free product by suitable compaction or granulation processes. This involves removing on the whole more than 90% of the water, so that the water content in the product is less than 10% by weight, less than 5% by weight, less than 4% by weight or less than 3% by weight.

Analysis of L-amino acids to determine the concentration at one or more time(s) during the fermentation may be carried out by separating the L-amino acids by means of ion exchange chromatography, preferably cation exchange chromatography, with subsequent post-column derivatization using ninhydrin, as described in Spackman et al. (Analytical Chemistry 30: 1190-1206 (1958)). It is also possible to employ *ortho*-phthalaldehyde rather than ninhydrin for post-column derivatization. An overview article on ion exchange chromatography can be found in Pickering (LC-GC (Magazine of Chromatographic Science) 7(6), 484-487 (1989)).

It is likewise possible to carry out a pre-column derivatization, for example by using *ortho*-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51: 1167-1174 (1979)).

Detection is carried out photometrically (absorbance, fluorescence).

A review regarding amino acid analysis can be found *inter alia* in the textbook "Bioanalytik" by Lottspeich and Zorbas (Spektrum Akademischer Verlag, Heidelberg, Germany 1998).

The present invention is furthermore illustrated by the figures and non-limiting examples below, from which further features, embodiments, aspects and advantages of the present invention may be drawn.

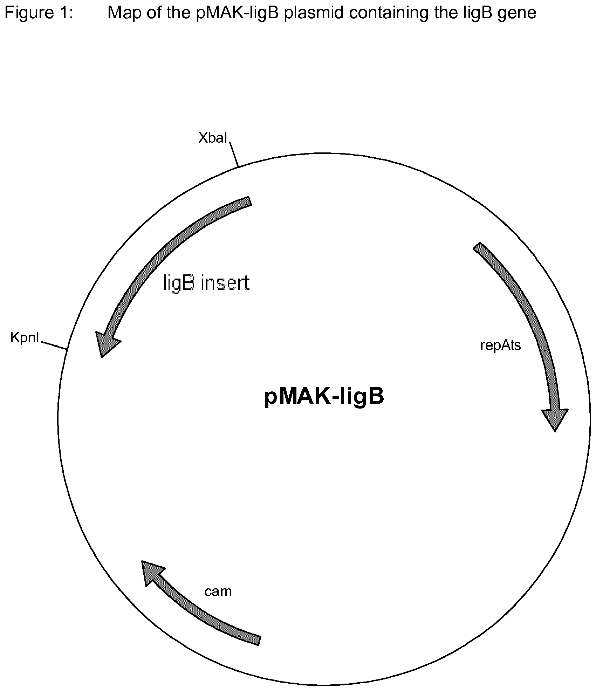

**Fig. 1** shows a map of plasmid pMAK-ligB which contains a section of the ligB gene.

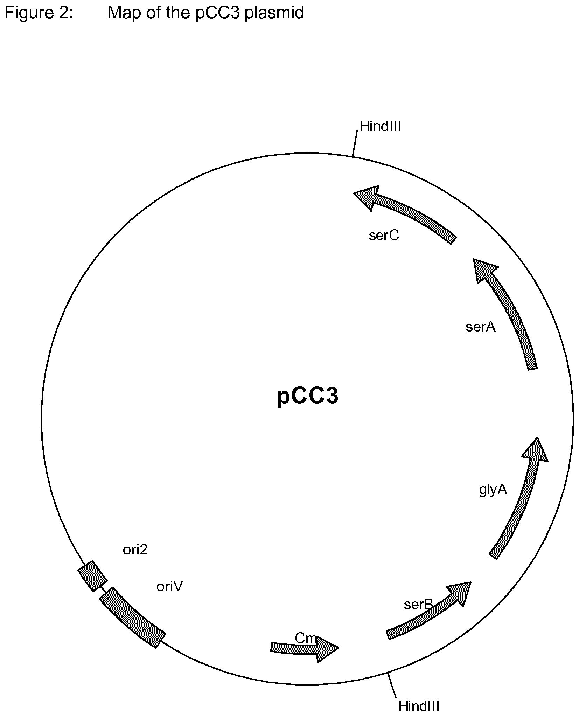

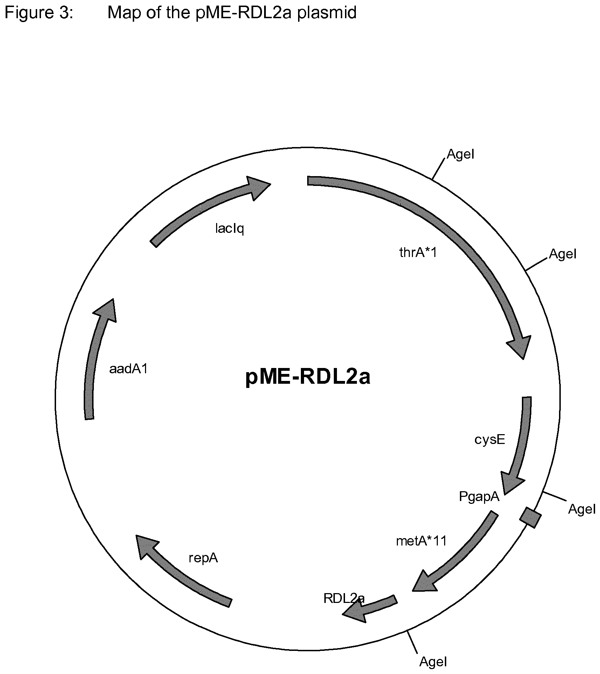

**Fig. 2** shows a map of plasmid pCC3.
**Fig**. **3** shows a map of plasmid pME-RDL2a.

Length information should be taken as approximate values. The abbreviations and reference names used have the following meaning:
- aadA1: streptomycin/spectinomycin-resistance gene
- laclq: gene for the trc-promoter repressor protein
- repAts: plasmid replication protein (ts); also repA
- oriV: origin of replication
- ori2: replication origin
- cam/Cm: chloramphenicol-resistance gene
- ligB insert: part of the coding region of the ligB gene
- serC: coding region of the serC gene
- serA: coding region of the serA gene
- glyA: coding region of the glyA gene
- serB: coding region of the serB gene
- thrA*1: coding region of the thrA gene (feedback-resistant allele)
- cysE: coding region of the cysE gene
- PgapA: gap promoter
- metA*11: coding region of the metA gene (feedback-resistant allele)
- RDL2a: coding region of the RDL2 allele coding for thiosulphate sulphurtransferase

The abbreviations for the restriction enzymes are defined as follows:
- Agel: restriction endonuclease from *Ruegeria gelatinovora*
- HindIII. restriction endonuclease from *Haemophilus influenzae* Rd
- Xbal: restriction endonuclease from *Xanthomonas campestris*
- Kpnl: restriction endonuclease from *Klebsiella pneumoniae*

The present invention is illustrated in more detail below on the basis of exemplary embodiments.

Minimal (M9) and complete media (LB) used for E. *coli* have been described by J.H. Miller (A Short Course in Bacteriol Genetics (1992), Cold Spring Harbor Laboratory Press). Isolation of plasmid DNA from *E. coli* and all techniques regarding restriction, ligation, Klenow- and alkaline-phosphatase treatment are carried out according to Sambrook et al. (Molecular Cloning - A Laboratory Manual (1989) Cold Spring Harbor Laboratory Press), unless stated otherwise. Transformation of *E. coli* is carried out according to Chung et al. (Proc. Natl. Acad. Sci. 86: 2172-2175, 1989), unless stated otherwise.

Unless stated otherwise, the incubation temperature for production of strains and transformants is 37°C.

### Example 1

### Cloning of pMAK-ligB

The plasmid pMAK-ligB was cloned as a selection marker for the transduction of spoT alleles and inserted into the ligB gene in the chromosome of *E. coli* DM1849. The ligB gene is located in the chromosome about 1.2 kbp upstream of spoT. The *E. coli* DM1849 strain has a spoT allele according to the invention.

The PCR primers ligB-up and ligB-down have on their 5' ends in each case 6 randomly selected nucleotides followed by recognition sequences for the restriction endonucleases XbaI (tctaga) and KpnI (ggtacc), respectively. Nucleotides 13 to 32 of ligB-up bind from positions 3817496 to 3817516 in the *E. coli* MG1655 genome. Nucleotides 13 to 32 of ligB-down bind from positions 3818519 to 3818498 in the *E. coli* MG1655 genome.
ligB-up (SEQ ID NO:9)
   5' CAGTACtctagaAGCCACGAAGGACACTAAGG 3'
ligB-down (SEQ ID NO:10)
   5' TTAGTTggtaccCGGATGGACCGCAGTTAATG 3'

These two primers and genomic DNA of *E. coli* MG1655 as template were used to carry out a PCR. The PCR product was 1045 bp in length and included the sequence from positions 3817496 to 3818519 of the MG1655 genome (SEQ ID NO:11).

Said PCR product was purified using a QIAquick PCR purification kit (Qiagen, Hilden, Germany), cleaved by the XbaI and KpnI restriction enzymes and purified again. Plasmid pMAK705 (Hamilton CM, Aldea M, Washburn BK, Babitzke P, Kushner SR (1989); J Bacteriol.; 171(9): 4617-4622) was cleaved by the XbaI and KpnI restriction enzymes, dephosphorylated by alkaline phosphatase (alkaline phosphatase, calf intestinal, New England Biolabs, Frankfurt a.M., Germany) and purified using a QIAquick PCR purification kit (Qiagen, Hilden). The PCR product was then ligated with pMAK705, and the ligation mix was transformed into *E. coli* DH5α. Correct plasmid clones were selected by 20 mg/l chloramphenicol and identified by restriction cleavage and subsequent sequencing of the inserts. The plasmid obtained in this way was named pMAK-ligB.

### Example 2

### Integration of pMAK-ligB into the donor strain DM1849

The *E. coli* strain DM1849 carries three mutations in the ppGppase-encoding spoT gene: an insertion of the six nucleotides CATGAT downstream of nucleotide position 252 (corresponding to position 3820674 in the MG1655 genome), the substitution g520t (based on the wild-type spoT gene, corresponding to position 3820942 in the MG1655 genome) and the substitution c1585t (based on the wild-type spoT gene, corresponding to position 3822007 in the MG1655 genome).

The DM1849 strain was transformed with plasmid pMAK-ligB by electroporation. pMAK-ligB has a chloramphenicol-resistance gene and a temperature-sensitive origin of replication. The plasmid is replicated by *E. coli* at 30°C but not at 44°C. The transformation mix was plated on LB agar containing 20 mg/l chloramphenicol and incubated at 30°C for 40 hours. Cells were then picked up using an inoculation loop, resuspended in LB medium and diluted 1 in 10 000 with LB medium. 100 µl of the dilution were plated on LB agar containing 20 mg/l chloramphenicol and incubated at 44°C for another 24 hours. As a result, colonies were selected where the pMAK-ligB plasmid was chromosomally integrated in the ligB gene. One of these colonies was thinned out using an inoculation loop on LB agar containing 20 mg/l chloramphenicol and incubated at 44°C for 24 hours. The resulting strain was named DM1849::pMAK-ligB.

### Example 3

### Generation of a P1-phage library from DM1849::pMAK-ligB

First, 10 ml of LB medium were inoculated with the DM1849::pMAK-ligB strain and cultured at 44°C for 18 hours. Subsequently, 100 µl of the culture were transferred to 10 ml of LB medium and cultured at 44°C to an optical density (at 600 nm) of 0.5. Then calcium chloride, up to a final concentration of 5 mM, and glucose, up to a final concentration of 2 g/l, were added. This was followed by adding 100 µl of a P1-phage suspension and incubating the cells at 37°C. After 4 hours, the culture was centrifuged and the supernatant was sterile-filtered twice.

### Example 4

### Transduction of the methionine producer E. coli ECM1 with the P1-phage library from DM1849::pMAK-ligB for transferring the three mutations of DM1849

The L-methionine-producing *E. coli* strain ECM1 carries a feedback-resistant metA allele, a deletion of the metJ gene and a copy of the strong trc promoter upstream of each of the genes metH, metF, cysP and cysJ. It is based on the wild-type K12 strain MG1655.

10 ml of LB medium were inoculated with the acceptor strain ECM1 and cultured at 37°C for 18 hours. This was followed by removing 2 ml of the culture by centrifugation and resuspending the cell pellet in 1 ml of LB medium containing 10 mM MgSO₄ and 5 mM CaCl₂. To 300 µl of the cell suspension 100 µl of the P1-phage library from DM1849::pMAK-ligB were added and the mixture was incubated at 37°C for 30 min. This was followed by adding 200 µl of 1 M trisodium citrate and vortexing briefly. Then 1 ml of LB medium was added and the cells were grown at 44°C for one hour. Subsequently, the cells were washed twice with in each case 1 ml of LB containing 100 mM trisodium citrate and finally resuspended in 100 µl of LB containing 100 mM trisodium citrate. They were streaked out on LB agar containing 20 mg/l chloramphenicol and incubated at 44°C for 48 hours. Ten colonies were thinned out on LB agar containing 20 mg/l chloramphenicol and incubated again at 44°C for 48 hours. In these clones, a genomic fragment had been transduced that comprised the ligB region including the integrated pMAK-ligB plasmid. By subsequent DNA sequencing of the relevant sections of the spoT gene, five clones were identified in which the spoT allele from DM1849 had been co-transduced.

Excision of the pMAK-ligB plasmid from the chromosome and curing were carried out as described in Hamilton *et al.* (CM Hamilton, Aldea M, Washburn BK, Babitzke P, Kushner SR (1989); J Bacteriol.; 171(9); 4617-4622). One of the plasmid-free clones obtained in this way was named ECM1_spoT1849.

The strains ECM1 and ECM1_spoT1849 were deposited according to the Budapest Treaty with the DSMZ (Deutsche Sammlung von Mikroorganismen und Zellkulturen GmbH, Inhoffenstraße 7B, 38124 Brunswick, Germany) under the DSM numbers DSM 25066 (= ECM1) and DSM 25067 (= EMC1_spoT1849) on 3 August 2011.

### Example 5

### Cloning of the serC gene into plasmid pUC18

The *E*. *coli* MG1655 serC gene was amplified with the aid of the polymerase chain reaction (PCR) and subsequently cloned into the pUC18 plasmid (Fermentas GmbH, St. Leon-Rot, Germany).

The PCR primers serCF(XbaI) and serCR(HindIII) have on their 5' ends in each case randomly selected nucleotides followed by recognition sequences for the restriction endonucleases XbaI (TCTAGA) and HindIII (AAGCTT), respectively. Nucleotides 13 to 38 of serCF(XbaI) bind from positions 956619 to 956644 in the *E. coli* MG1655 genome. Nucleotides 13 to 37 of serCR(HindIII) bind from positions 958028 to 958004 in the *E*. *coli* MG1655 genome.
serCF(XbaI) (SEQ ID NO: 12)
   5' AGGTGCTCTAGAGTCCGCGCTGTGCAAATCCAGAATGG 3'
serCR(HindIII) (SEQ ID NO: 13)
   5' TACACCAAGCTTAACTCTCTACAACAGAAATAAAAAC 3'

The serC gene was amplified by polymerase chain reaction (PCR) using the serCF(XbaI) and serCR(HindIII) primers and Phusion DNA polymerase (Finnzymes Oy, Espoo, Finland). Genomic DNA of *E*. *coli* MG1655 served as template. The resulting DNA fragment was 1434 bp in size. It was cleaved by the XbaI and HindIII restriction endonucleases and purified with the aid of a QIAquick PCR purification kit (Qiagen, Hilden, Germany). Non-methylated pUC18 plasmid DNA was cleaved by the Xbal and HindIII restriction endonucleases and purified with the aid of a QIAquick PCR purification kit (Qiagen, Hilden, Germany). The cleaved plasmid was then ligated with the PCR product and transformed into *E*. *coli* DH5α. Plasmid clones containing the serC gene were identified by restriction cleavage and DNA sequencing. The resulting plasmid was named pUC18-serC.

### Example 6

### Cloning of the serA gene into plasmid pUC18-serC

The *E*. *coli* MG1655 serA gene was amplified with the aid of the polymerase chain reaction (PCR) and subsequently cloned into the pUC18-serC plasmid.

The PCR primer serAF(XbaI) has on its 5' end 6 randomly selected nucleotides followed by a recognition sequence for the restriction endonuclease Xbal (TCTAGA). Nucleotides 12 to 33 bind from positions 3055199 to 3055220 in the *E. coli* MG1655 genome. The PCR primer serAR(SHSSNB) has on its 5' end 6 randomly selected nucleotides followed by recognition sequences for the restriction endonucleases SacI, HindIII, SphI, SmaI, NotI and BglII. Nucleotides 49 to 58 bind from positions 3056880 to 3056861 in the *E*. *coli* MG1655 genome.
serAF(XbaI) (SEQ ID NO: 14)
   5' CTGTAGTCTAGATTAGTACAGCAGACGGGCGCG 3'
serAR(SHSSNB) (SEQ ID NO: 15)

5'
CAAGAGCTCAAGCTTGCATGCGATTCCCGGGCGGCCGCAATAAGATCTCCGTC
AGGGCGTGGTGACCG 3'

The serA gene was amplified by polymerase chain reaction (PCR) using the serAF(XbaI) and serAR(SHSSNB) primers and Phusion DNA polymerase (Finnzymes Oy, Espoo, Finland). Genomic DNA of *E*. *coli* MG1655 served as template. The resulting DNA fragment was 1731 bp in size.

It was cleaved by the XbaI and SacI restriction endonucleases and purified with the aid of a QIAquick PCR purification kit (Qiagen, Hilden, Germany). The pUC18-serC plasmid was likewise cleaved by the Xbal and SacI restriction endonucleases and purified with the aid of a QIAquick PCR purification kit (Qiagen, Hilden, Germany). The cleaved plasmid was then ligated with the PCR product and transformed into *E*. *coli* DH5α. Plasmid clones containing the serA gene were identified by restriction cleavage and DNA sequencing. The resulting plasmid was named pUC18-serAC.

### Example 7

### Cloning of the serB gene into plasmid pUC18-serAC

The *E*. *coli* MG1655 serB gene was amplified with the aid of the polymerase chain reaction (PCR) and subsequently cloned into the pUC18-serAC plasmid.

The PCR primer serB(SphI) has on its 5' end 6 randomly selected nucleotides followed by a recognition sequence for the restriction endonuclease Sphl (GCATGC). Nucleotides 13 to 34 bind from positions 4622816 to 4622837 in the *E*. *coli* M1655 genome.

The PCR primer serB(SmaI) has on its 5' end 6 randomly selected nucleotides followed by recognition sequences for the restriction endonucleases SalI (GTCGAC) and SmaI (CCCGGG). Nucleotides 54 to 75 bind from positions 4623887 to 4623866 in the *E*. *coli* MG1655 genome.
serB(SphI) (SEQ ID NO: 16)
   5' CCATGCGCATGCCCACCCTTTGAAAATTTGAGAC 3'
serB(SmaI) (SEQ ID NO: 17)

5' CCGCATGTCGACATCCCGGGGCAGAAAGGCCCACCCGAAGGTGAGCCAGTGTG
ATTACTTCTGATTCAGGCTGCC 3'

The serB gene was amplified by polymerase chain reaction (PCR) using the serB(SphI) and serB(SmaI) primers and Phusion DNA polymerase (Finnzymes Oy, Espoo, Finland). Genomic DNA of *E*. *coli* MG1655 served as template. The resulting DNA fragment was 1137 bp in size.

It was cleaved by the SphI and Smal restriction endonucleases and purified with the aid of a QIAquick PCR purification kit (Qiagen, Hilden, Germany). The pUC18-serAC plasmid was likewise cleaved by the Sphl and Smal restriction endonucleases, dephosphorylated by alkaline phosphatase and purified with the aid of a QIAquick PCR purification kit (Qiagen, Hilden, Germany). The cleaved plasmid was then ligated with the PCR product and transformed into *E*. *coli* DH5α. Plasmid clones containing the serB gene were identified by restriction cleavage and DNA sequencing. The resulting plasmid was named pUC18-serBAC.

### Example 8

### Cloning of the glyA gene into plasmid pUC18-serBAC

The *E*. *coli* MG1655 glyA gene was amplified with the aid of the polymerase chain reaction (PCR) and subsequently cloned into the pUC18-serBAC plasmid.

The PCR primer glyA-downstream has on its 5' end 6 randomly selected nucleotides followed by a recognition sequence for the restriction endonuclease BglII (AGATCT). Nucleotides 13 to 35 bind from positions 2682063 to 2682085 in the *E. coli* MG1655 genome.

The PCR primer glyA-upstream has on its 5' end randomly selected nucleotides followed by recognition sequences for the restriction endonuclease NotI (GCGGCCGC). Nucleotides 15 to 33 bind from positions 2683762 to 2683744 in the *E*. *coli* M1655 genome.
glyA-downstream(SEQ ID NO: 18)
   5' ATCTAAAGATCTGTTACGACAGATTTGATGGCGCG 3'
glyA-upstream (SEQ ID NO: 19)
   5' TTCATCGCGGCCGCGAAAGAATGTGATGAAGTG 3'

The glyA gene was amplified by polymerase chain reaction (PCR) using the glyA-downstream and glyA-upstream primers and Phusion DNA polymerase (Finnzymes Oy, Espoo, Finland). Genomic DNA of *E. coli* MG1655 served as template. The resulting DNA fragment was 1726 bp in size.

It was cleaved by the BglII and Notl restriction endonucleases and purified with the aid of a QIAquick PCR purification kit (Qiagen, Hilden, Germany). The pUC18-serBAC plasmid was likewise cleaved by the BglII and NotI restriction endonucleases and purified with the aid of a QIAquick PCR purification kit (Qiagen, Hilden, Germany). The cleaved plasmid was then ligated with the PCR product and transformed into *E*. *coli* DH5α. Plasmid clones containing the glyA gene were identified by restriction cleavage and DNA sequencing. The resulting plasmid was named pUC18-serB-glyA-serAC.

### Example 9

### Cloning of the genes serB-glyA-serAC from pUC18-serB-glyA-serAC to pCC1-BAC

The pUC18-serB-glyA-serAC plasmid was cleaved by the HindIII restriction endonuclease and the DNA fragments were fractionated by agarose gel electrophoresis. A 5.9 kb DNA fragment containing the serB, glyA, serA and serC genes was isolated from the gel. The fragment was ligated with the plasmid pCC1BAC Cloning-Ready Vector (Hind III) from Epicentre /Madison, USA, which had previously been cleaved by HindIII, and transformed to *E. coli* EPI300. Plasmid clones containing the DNA fragment of serB, glyA, serA and serC were identified by restriction cleavage and DNA sequencing. The resulting production plasmid was named pCC3.

### Example 10

### Cloning of the production plasmid pME-RDL2a

Cloning of the pME-RDL2a production plasmid was carried out as described in EP application 11151526.8. It includes the *E. coli* cysE gene, feedback-resistant alleles of the *E*. *coli* thrA and metA genes and the RDL2a gene which codes for the *Saccharomyces cerevisiae* thiosulphate sulphurtransferase RDL2p. In addition, it includes a streptomycin-resistance gene.

### Example 11

### Transformation of strains ECM1 and ECM1 spoT1849 with the production plasmids

The strains ECM1 and ECM1_spoT1849 were transformed with the production vector pCC3 of Example 9, and the transformants were selected using 20 mg/l chloramphenicol. The cells were then transformed with plasmid pME-RDL2a of Example 10 and the resulting transformants were selected using 20 mg/l chloramphenicol + 100 mg/l streptomycin. The resulting strains were named ECM1/pCC3/pME-RDL2a and ECM1_spoT1849/pCC3/pME-RDL2a.

### Example 12

### Performance assay in a shaker-flask experiment

Performance of the *E. coli* L-methionine producer strains was evaluated by production tests in 100 ml conical flasks. Precultures of in each case 10 ml of preculture medium (10% LB medium containing 2.5 g/l glucose and 90% PC1 minimal medium) inoculated with 100 µl of cell culture were grown at 37°C for 10 hours. These were then used to inoculated in each case 10 ml of PC1 minimal medium (Table 1) to an OD 600 nm of 0.2 (Eppendorf Bio-Photometer; Eppendorf AG, Hamburg, Germany) and the cultures were grown at 37°C for 24 hours. The extracellular L-methionine concentration was determined by ion exchange chromatography and post-column derivatization with ninhydrin detection using an amino acid analyser (Sykam GmbH, Eresing, Germany). The extracellular glucose concentration was determined using a YSI 2700 Select Glucose Analyzer (YSI Life Sciences, Yellow Springs, Ohio, USA). The results are listed in Table 2. After 24 hours glucose had been used up completely in both cultures. The methionine concentration in the culture supernatant of ECM1_spoT1849/pCC3/pME-RDL2a was, at 1.56 g/l, distinctly higher than in the comparative strain (1.01 g/l). Modification of the gene coding for ppGppase by way of introducing the mutations Gly174, Leu529 and an insertion between Asp84 and Met85 thus resulted in an increase in the methionine yield over the starting strain from 10.1% (grams of methionine per gram of glucose) to 15.6%.

**Table 1: Minimal medium PC1**

| Substance | Concentration |
|---|---|
| ZnSO4 * 7 H2O | 4 mg/l |
| CuCl2 * 2 H2O | 2 mg/l |
| MnSO4 * H2O | 20 mg/l |
| H3BO3 | 1 mg/l |
| Na2MoO4 * 2 H2O | 0.4 mg/l |
| MgSO4 * 7 H2O | 1 g/l |
| Citric acid * 1 H2O | 6.56 g/l |
| CaCl2 * 2 H2O | 40 mg/l |
| K2HPO4 | 8.02 g/l |
| Na2HPO4 | 2 g/l |
| (NH4)2HPO4 | 8 g/l |
| NH4Cl | 0.13 g/l |
| (NH4)2SO3 | 5.6 g/l |
| MOPS | 5 g/l |
| NaOH 10M | adjusted to pH 6.8 |
| | |
| FeSO4 * 7 H2O | 40 mg/l |
| Thiamine hydrochloride | 10 mg/l |
| Vitamin B12 | 10 mg/l |
| Glucose | 10 g/l |
| Isopropyl-thio-ß-galactoside (IPTG) | 2.4 mg/l |
| Spectinomycin | 50 mg/l |
| Chloramphenicol | 20 mg/l |

**Table 2: L-Methionine concentrations in the fermentation broths of E. coli strains ECM1/pCC3/pME-RDL2a and ECM1_spoT1849/pCC3/pME-RDL2a**

| Strain | OD(600nm) | L-Methionine (g/l) |
|---|---|---|
| ECM1/pCC3/pME-RDL2a | 6.36 | 1.01 |
| ECM1_spoT1849/pCC3/pME-RDL2a | 7.46 | 1.56 |

The features of the invention disclosed in the foregoing description and in the claims may both individually and in any combination be essential to implementing the invention in its various embodiments.

## Claims

1. Method of preparing methionine or tryptophan, comprising the step of culturing a *E. coli* cell, which already overproduces methionine or tryptophan and is further genetically modified over its wild type in such a way that it has a reduced ppGppase activity relative to its wild type, wherein the *E*. *coli* cell has a (p)ppGpp-synthetase activity which is essentially unchanged relative to its wild type and wherein the ppGppase is at least one selected from the group comprising the amino acid sequences SEQ ID NO:2, SEQ ID NO:4 and SEQ ID NO:6.

2. Method according to claim 1, wherein the activity in the cultured *E*. *coli* cell of at least one ppGppase from the group comprising the amino acid sequences SEQ ID NO:2, SEQ ID NO:4 and SEQ ID NO:6 is reduced as a result of said ppGppase having at least the following modification: an insertion of the two amino acids His and Asn is present between Asp84 and Met85.

3. Method according to claim 1 or 2, further comprising the step of purifying the essential amino acid.

4. *E. coli* cell, which already overproduces methionine or tryptophan, wherein the activity of at least one ppGppase from the group comprising the amino acid sequences SEQ ID NO:2, SEQ ID NO:4 and SEQ ID NO:6 is reduced as a result of said ppGppase having the following modification: an insertion of the two amino acids His and Asn is present between Asp84 and Met85, and wherein the *E*. *coli* cell has a (p)ppGpp-synthetase activity which is essentially unchanged relative to its wild type.

5. *E. coli* cell as claimed in claim 4 wherein the activity of at least one ppGppase comprising the amino acid sequence SEQ ID NO:2 is reduced as a result of said ppGppase having the following modifications: an insertion of the two amino acids His and Asn is present between Asp84 and Met85 and wherein the *E*. *coli* cell has a (p)ppGpp-synthetase activity which is essentially unchanged relative to its wild type.

6. *E. coli* cell according to Claim 5, wherein the ppGppase additionally has at least one modification from the group comprising substitutionsat the amino acids Gln9, Thr13, Tyr63, Arg109, Gln225, Cys245, Val248, Asn268, Ser270, Met280, His344, Pro436, Asn501, Gln505, His543, Ala546, Ser547, Ile548, His555, Gly556, His557, Pro559, Lys619, Thr621, Ala622, Thr627, Thr651, Ala669, Ala675, Thr698 and an insertion between Glu343 and His344.

7. *E. coli* cell according to either of Claims 4 to 6, wherein the ppGppase has at least one modification from the group having the following substitutions:
1.) substitution of Gln9 by an amino acid selected from the group consisting of Leu, Ile and Val,
2.) substitution of Thr13 by an amino acid selected from the group consisting of Lys, Arg, His, Gln and Asn,
3.) substitution of Tyr63 by an amino acid selected from the group consisting of Lys, Arg and His,
4.) substitution of Arg109 by an amino acid selected from the group consisting of Gln and Asn,
5.) substitution of Gln225 by an amino acid selected from the group consisting of Ser, Ala and Thr,
6.) substitution of Cys245 by an amino acid selected from the group consisting of Leu, Ile and Val,
7.) substitution of Val248 by an amino acid selected from the group consisting of Lys, Arg and His,
8.) substitution of Asn268 by an amino acid selected from the group consisting of Lys, Arg and His,
9.) substitution of Ser270 by an amino acid selected from the group consisting of Ala, Leu, Ile and Val,
10.) substitution of Met280 by an amino acid selected from the group consisting of Ser, Thr and Ala,
11.) insertion of the amino acids Lys and Glu between amino acids Glu343 and His344,
12.) substitution of His344 by an amino acid selected from the group consisting of Gln and Asn,
13.) substitution of Pro436 by an amino acid selected from the group consisting of Ser, Ala and Thr,
14.) substitution of Asn501 by an amino acid selected from the group consisting of Ser, Ala and Thr,
15.) substitution of Gln505 by an amino acid selected from the group consisting of Pro, Ser, Ala and Thr,
16.) substitution of His543 by an amino acid selected from the group consisting of Asn and Gln,
17.) substitution of Ala546 by an amino acid selected from the group consisting of Asn and Gln,
18.) substitution of Ser547 by an amino acid selected from the group consisting of Ala, Leu, Ile and Val,
19.) substitution of Ile548 by an amino acid selected from the group consisting of Asn and Gln,
20.) substitution of His555 by an amino acid selected from the group consisting of Leu, Ile and Val,
21.) substitution of glycine in position 556 by Lys, Arg and His,
22.) substitution of His557 by an amino acid selected from the group consisting of Asn and Gln,
23.) substitution of Pro559 by an amino acid selected from the group consisting of Ser, Ala and Thr,
24.) substitution of Lys619 by an amino acid selected from the group consisting of Asn and Gln,
25.) substitution of Thr621 by an amino acid selected from the group consisting of Leu, Ile and Val,
26.) substitution of Ala622 by an amino acid selected from the group consisting of Glu and Asp,
27.) substitution of Thr627 by an amino acid selected from the group consisting of Ala and Gly,
28.) substitution of Thr651 by an amino acid selected from the group consisting of Glu and Asp,
29.) substitution of Ala669 and/or Ala675 by Thr,
30.) substitution of Thr698 by an amino acid selected from the group consisting of Gln and Asn.

8. *E. coli* cell according to any of Claims 4 to 7, wherein the *E*. *coli* cell overexpresses, relative to its wild type, at least one nucleic acid sequence that codes for any of the following enzymes
1.) thiosulphate/sulphate transport system CysPUWA (EC 3.6.3.25),
2.) 3'-phosphoadenosine 5'-phosphosulphate reductase CysH (EC 1.8.4.8),
3.) sulphite reductase CysJI (EC 1.8.1.2),
4.) cysteine synthase A CysK (EC 2.5.1.47),
5.) cysteine synthase B CysM (EC 2.5.1.47),
6.) serine acetyltransferase CysE (EC 2.3.1.30),
7.) glycine cleavage system GcvTHP-Lpd (EC 2.1.2.10, EC 1.4.4.2, EC 1.8.1.4),
8.) lipoyl synthase LipA (EC 2.8.1.8),
9.) lipoyl-protein ligase LipB (EC 2.3.1.181),
10.) phosphoglycerate dehydrogenase SerA (EC 1.1.1.95),
11.) 3-phosphoserine phosphatase SerB (EC 3.1.3.3),
12.) 3-phosphoserine/phosphohydroxythreonine aminotransferase SerC (EC 2.6.1.52),
13.) serine hydroxymethyltransferase GlyA (EC 2.1.2.1),
14.) aspartokinase I and homoserine dehydrogenase I ThrA (EC 2.7.2.4, EC 1.1.1.3),
15.) aspartate kinase LysC (EC 2.7.2.4),
16.) homoserine dehydrogenase Hom (EC 1.1.1.3),
17.) homoserine O-acetyltransferase MetX (EC 2.3.1.31),
18.) homoserine O-succinyltransferase MetA (EC 2.3.1.46),
19.) cystathionine gamma-synthase MetB (EC 2.5.1.48),
20.) C-S lyase AecD (EC 4.4.1.8, also referred to as beta-lyase),
21.) cystathionine beta-lyase MetC (EC 4.4.1.8),
22.) B12-independent homocysteine S-methyltransferase MetE (EC 2.1.1.14),
23.) B12-dependent homocysteine S-methyltransferase MetH (EC 2.1.1.13),
24.) methylenetetrahydrofolate reductase MetF (EC 1.5.1.20),
25.) L-methionine exporter BrnFE from Corynebacterium glutamicum,
26.) valine exporter YgaZH from Escherichia coli (b2682, b2683),
27.) putative transporter YjeH from Escherichia coli (b4141),
28.) pyridine nucleotide transhydrogenase PntAB (EC 1.6.1.2),
29.) O-succinylhomoserine sulphhydrylase MetZ (EC 2.5.1.48),
30.) phosphoenolpyruvate carboxylase Pyc (EC 4.1.1.31),
31.) thiosulphate sulphurtransferase RDL2p (EC 2.8.1.1),
32.) thiosulphate-thiol sulphurtransferase (EC 2.8.1.3),
33.) thiosulphate-dithiol sulphurtransferase (EC 2.8.1.5).

9. *E. coli* cell according to any of Claims 4 to 8, wherein the *E*. *coli* cell expresses on a reduced scale, relative to its wild type, at least one nucleic acid sequence that codes for any of the following enzymes:
1.) transcriptional regulator of L-methionine biosynthesis (MetJ) (b3938, ECK3930),
2.) glucose-6-phosphate isomerase (Pgi, EC 5.3.1.9) (b4025, ECK4017), 3.) homoserine kinase (ThrB, EC 2.7.1.39) (b0003, ECK0003),
4.) S-adenosylmethionine synthase (MetK, EC 2.5.1.6) (b2942, ECK2937), 5.) dihydrodipicolinate synthase (DapA, EC 4.2.1.52) (b2478, ECK2474),
6.) phosphoenolpyruvate carboxykinase (Pck, EC 4.1.1.49) (b3403, ECK3390),
7.) formyltetrahydrofolate hydrolase (PurU, EC 3.5.1.10) (b1232, ECK1227),
8.) pyruvate kinase II (PykA, EC 2.7.1.40) (b1854, ECK1855),
9.) pyruvate kinase I (PykF, EC 2.7.1.40) (b1676, ECK1672),
10.) subunit of L-methionine transporter (MetQNI) (b0197, ECK0197),
11.) subunit of L-methionine transporter (MetQNI) (b0198, ECK0198),
12.) subunit of L-methionine transporter (MetQNI) (b0199, ECK0199),
13.) deoxycytidine 5'-triphosphate deaminase (Dcd, EC 3.5.4.13) (b2065, ECK2059),
14.) putative N-acyltransferase (YncA), 15.) regulatory sRNA FnrS,
16.) sigma factor RpoS.

10. *E. coli* cell according to any of Claims 4 to 9, wherein the *E*. *coli* cell overexpresses, relative to its wild type, at least one nucleic acid sequence that codes for any of the following enzymes:
1.) anthranilate synthase (trpDE, EC 4.1.3.27), anthranilate phosphoribosyltransferase (trpD, EC 2.4.2.18), phosphoribosylanthranilate isomerase (trpC, EC 5.3.1.24), indole-3-glycerol-phosphate synthase (trpC, EC 4.1.1.48) and tryptophan synthase (trpAB, EC 4.1.2.8 and 4.2.1.122),
2.) phosphoglycerate dehydrogenase SerA (EC 1.1.1.95),
3.) 3-phosphoserine phosphatase SerB (EC 3.1.3.3),
4.) 3-phosphoserine/phosphohydroxythreonine aminotransferase SerC (EC 2.6.1.52),
5.) L-tyrosine-sensitive DHAP synthase (aroF, EC 2.5.1.54),
6.) L-phenylalanine feedback-resistant DHAP synthase (aroG, EC 2.5.1.54), 7.) L-tryptophan-sensitive DHAP synthase (aroH, EC 2.5.1.54),
8.) phosphoenolpyruvate synthase ppsA (EC 2.7.9.2),
9.) phosphoenolpyruvate carboxykinase pck (EC 4.1.1.49), 10.) transketolase A tktA (EC 2.2.1.1),
11.) transketolase B tktB (EC 2.2.1.1),
12.) gene product of the *E*. *coli* open reading frame (ORF) yddG.

11. *E*. *coli* cell according to any of Claims 4 to 10, wherein the *E*. *coli* cell expresses on a reduced scale, relative to its wild type, at least one nucleic acid sequence that codes for any of the following enzymes:
1.) tryptophanase (tnaA, EC 4.1.99.1),
2.) repressor of the trp operon (trpR),
3.) chorismate mutase T or prephenate dehydrogenase (tyrA, EC 1.3.1.12), 4.) chorismate mutase P or prephenate dehydrogenase (pheA, EC 4.2.1.51),
5.) tryptophan-specific transport protein (mtr), 6.) tryptophan permease (tnaB),
7.) transporter for aromatic amino acids (aroP),
8.) L-serine deaminase (sdaA, EC 4.3.1.17),
9.) glucose-6-phosphate isomerase (pgi, EC 5.3.1.9), 10.) tyrosine aminotransferase (tyrB),
11.) repressor of the glp regulon (glpR);
12.) sigma factor RpoS (rpoS).

12. Method of preparing methionine or tryptophan, comprising the step of culturing a *E. coli* cell as defined in any of claims 4 to 11.

## Patentansprüche

1. Verfahren zur Herstellung von Methionin oder Tryptophan, umfassend den Schritt des Kultivierens einer *E. coli*-Zelle, die bereits Methionin oder Tryptophan überproduziert und ferner ihrem Wildtyp gegenüber derartig gentechnisch verändert ist, dass sie eine relativ zu ihrem Wildtyp verringerte ppGppase-Aktivität aufweist, wobei die *E. coli-*Zelle eine relativ zu ihrem Wildtyp im Wesentlichen unveränderte (p)ppGpp-Synthetase-Aktivität aufweist und wobei es sich bei der ppGppase um wenigstens eine handelt, die aus der die Aminosäuresequenzen SEQ ID NO:2, SEQ ID NO:4 und SEQ ID NO:6 umfassenden Gruppe ausgewählt ist.

2. Verfahren nach Anspruch 1, wobei die Aktivität wenigstens einer ppGppase aus der die Aminosäuresequenzen SEQ ID NO:2, SEQ ID NO:4 und SEQ ID NO:6 umfassenden Gruppe in der kultivierten *E. coli-*Zelle dadurch verringert ist, dass die ppGppase wenigstens die folgende Modifikation aufweist: zwischen Asp84 und Met85 liegt eine Insertion der beiden Aminosäuren His und Asn vor.

3. Verfahren nach Anspruch 1 oder 2, ferner umfassend den Schritt des Aufreinigens der essentiellen Aminosäure.

4. *E*. *coli*-Zelle, die bereits Methionin oder Tryptophan überproduziert, wobei die Aktivität wenigstens einer ppGppase aus der die Aminosäuresequenzen SEQ ID NO:2, SEQ ID NO:4 und SEQ ID NO:6 umfassenden Gruppe dadurch verringert ist, dass die ppGppase die folgende Modifikation aufweist: zwischen Asp84 und Met85 liegt eine Insertion der beiden Aminosäuren His und Asn vor, und wobei die *E. coli*-Zelle eine relativ zu ihrem Wildtyp im Wesentlichen unveränderte (p)ppGpp-Synthetase-Aktivität aufweist.

5. *E*. *coli*-Zelle gemäß Anspruch 4, wobei die Aktivität wenigstens einer die Aminosäuresequenz SEQ ID NO:2 umfassenden ppGppase dadurch verringert ist, dass die ppGppase die folgenden Modifikationen aufweist: zwischen Asp84 und Met85 liegt eine Insertion der beiden Aminosäuren His und Asn vor, und wobei die *E. coli*-Zelle eine relativ zu ihrem Wildtyp im Wesentlichen unveränderte (p)ppGpp-Synthetase-Aktivität aufweist.

6. *E*. *coli*-Zelle nach Anspruch 5, wobei die ppGppase zusätzlich wenigstens eine Modifikation aus der Gruppe aufweist, die Austausche an den Aminosäuren Gln9, Thr13, Tyr63, Arg109, Gln225, Cys245, Val248, Asn268, Ser270, Met280, His344, Pro436, Asn501, Gln505, His543, Ala546, Ser547, Ile548, His555, Gly556, His557, Pro559, Lys619, Thr621, Ala622, Thr627, Thr651, Ala669, Ala675, Thr698 und eine Insertion zwischen Glu343 und His344 umfasst.

7. *E. coli*-Zelle nach einem der Ansprüche 4 bis 6, wobei die ppGppase wenigstens eine Modifikation aus der Gruppe mit den folgenden Austauschen aufweist:
1.) Austausch von Gln9 gegen eine aus der aus Leu, Ile und Val bestehenden Gruppe ausgewählte Aminosäure,
2.) Austausch von Thr13 gegen eine aus der aus Lys, Arg, His, Gln und Asn bestehenden Gruppe ausgewählte Aminosäure,
3.) Austausch von Tyr63 gegen eine aus der aus Lys, Arg und His bestehenden Gruppe ausgewählte Aminosäure,
4.) Austausch von Arg109 gegen eine aus der aus Gln und Asn bestehenden Gruppe ausgewählte Aminosäure,
5.) Austausch von Gln225 gegen eine aus der aus Ser, Ala und Thr bestehenden Gruppe ausgewählte Aminosäure,
6.) Austausch von Cys245 gegen eine aus der aus Leu, Ile und Val bestehenden Gruppe ausgewählte Aminosäure,
7.) Austausch von Val248 gegen eine aus der aus Lys, Arg und His bestehenden Gruppe ausgewählte Aminosäure,
8.) Austausch von Asn268 gegen eine aus der aus Lys, Arg und His bestehenden Gruppe ausgewählte Aminosäure,
9.) Austausch von Ser270 gegen eine aus der aus Ala, Leu, Ile und Val bestehenden Gruppe ausgewählte Aminosäure,
10.) Austausch von Met280 gegen eine aus der aus Ser, Thr und Ala bestehenden Gruppe ausgewählte Aminosäure,
11.) Insertion der Aminosäuren Lys und Glu zwischen Aminosäuren Glu343 und His344,
12.) Austausch von His344 gegen eine aus der aus Gln und Asn bestehenden Gruppe ausgewählte Aminosäure,
13.) Austausch von Pro436 gegen eine aus der aus Ser, Ala und Thr bestehenden Gruppe ausgewählte Aminosäure,
14.) Austausch von Asn501 gegen eine aus der aus Ser, Ala und Thr bestehenden Gruppe ausgewählte Aminosäure,
15.) Austausch von Gln505 gegen eine aus der aus Pro, Ser, Ala und Thr bestehenden Gruppe ausgewählte Aminosäure,
16.) Austausch von His543 gegen eine aus der aus Asn und Gln bestehenden Gruppe ausgewählte Aminosäure,
17.) Austausch von Ala546 gegen eine aus der aus Asn und Gln bestehenden Gruppe ausgewählte Aminosäure,
18.) Austausch von Ser547 gegen eine aus der aus Ala, Leu, Ile und Val bestehenden Gruppe ausgewählte Aminosäure,
19.) Austausch von Ile548 gegen eine aus der aus Asn und Gln bestehenden Gruppe ausgewählte Aminosäure,
20.) Austausch von His555 gegen eine aus der aus Leu, Ile und Val bestehenden Gruppe ausgewählte Aminosäure,
21.) Austausch von Glycin in Position 556 gegen Lys, Arg und His,
22.) Austausch von His557 gegen eine aus der aus Asn und Gln bestehenden Gruppe ausgewählte Aminosäure,
23.) Austausch von Pro559 gegen eine aus der aus Ser, Ala und Thr bestehenden Gruppe ausgewählte Aminosäure,
24.) Austausch von Lys619 gegen eine aus der aus Asn und Gln bestehenden Gruppe ausgewählte Aminosäure,
25.) Austausch von Thr621 gegen eine aus der aus Leu, Ile und Val bestehenden Gruppe ausgewählte Aminosäure,
26.) Austausch von Ala622 gegen eine aus der aus Glu und Asp bestehenden Gruppe ausgewählte Aminosäure,
27.) Austausch von Thr627 gegen eine aus der aus Ala und Gly bestehenden Gruppe ausgewählte Aminosäure,
28.) Austausch von Thr651 gegen eine aus der aus Glu und Asp bestehenden Gruppe ausgewählte Aminosäure,
29.) Austausch von Ala669 und/oder Ala675 gegen Thr,
30.) Austausch von Thr698 gegen eine aus der aus Gln und Asn bestehenden Gruppe ausgewählte Aminosäure.

8. *E*. *coli*-Zelle nach einem der Ansprüche 4 bis 7, wobei die *E. coli*-Zelle relativ zu ihrem Wildtyp wenigstens eine Nukleinsäuresequenz überexprimiert, die für eines der folgenden Enzyme codiert:
1.) Thiosulfat/Sulfat-Transportsystem CysPUWA (EC 3.6.3.25),
2.) 3'-Phosphoadenosin-5'-phosphosulfat-Reduktase CysH (EC 1.8.4.8),
3.) Sulfit-Reduktase CysJI (EC 1.8.1.2),
4.) Cystein-Synthase A CysK (EC 2.5.1.47),
5.) Cystein-Synthase B CysM (EC 2.5.1.47),
6.) Serin-Acetyltransferase CysE (EC 2.3.1.30),
7.) Glycinspaltungssystem GcvTHP-Lpd (EC 2.1.2.10, EC 1.4.4.2, EC 1.8.1.4),
8.) Lipoyl-Synthase LipA (EC 2.8.1.8),
9.) Lipoyl-Protein-Ligase LipB (EC 2.3.1.181),
10.) Phosphoglycerat-Dehydrogenase SerA (EC 1.1.1.95),
11.) 3-Phosphoserin-Phosphatase SerB (EC 3.1.3.3),
12.) 3-Phosphoserin/Phosphohydroxythreonin-Aminotransferase SerC (EC 2.6.1.52),
13.) Serin-Hydroxymethyltransferase GlyA (EC 2.1.2.1),
14.) Aspartokinase I und Homoserin-Dehydrogenase I ThrA (EC 2.7.2.4, EC 1.1.1.3),
15.) Aspartatkinase LysC (EC 2.7.2.4),
16.) Homoserin-Dehydrogenase Hom (EC 1.1.1.3),
17.) Homoserin-O-Acetyltransferase MetX (EC 2.3.1.31),
18.) Homoserin-O-Succinyltransferase MetA (EC 2.3.1.46),
19.) Cystathionin-gamma-Synthase MetB (EC 2.5.1.48),
20.) C-S-Lyase AecD (EC 4.4.1.8, auch als Beta-Lyase bezeichnet),
21.) Cystathionin-beta-Lyase MetC (EC 4.4.1.8),
22.) B12-unabhängige Homocystein-S-Methyltransferase MetE (EC 2.1.1.14),
23.) B12-abhängige Homocystein-S-Methyltransferase MetH (EC 2.1.1.13),
24.) Methylentetrahydrofolat-Reduktase MetF (EC 1.5.1.20),
25.) L-Methionin-Exporter BrnFE aus Corynebacterium glutamicum,
26.) Valinexporter YgaZH aus Escherichia coli (b2682, b2683),
27.) putativer Transporter YjeH aus Escherichia coli (b4141),
28.) Pyridinnukleotid-Transhydrogenase PntAB (EC 1.6.1.2),
29.) O-Succinylhomoserin-Sulfhydrylase MetZ (EC 2.5.1.48),
30.) Phosphoenolpyruvat-Carboxylase Pyc (EC 4.1.1.31),
31.) Thiosulfat-Schwefeltransferase RDL2p (EC 2.8.1.1),
32.) Thiosulfat-Thiol-Schwefeltransferase (EC 2.8.1.3),
33.) Thiosulfat-Dithiol-Schwefeltransferase (EC 2.8.1.5).

9. *E*. *coli*-Zelle nach einem der Ansprüche 4 bis 8, wobei die *E. coli*-Zelle relativ zu ihrem Wildtyp wenigstens eine Nukleinsäuresequenz in einem verringerten Ausmaß exprimiert, die für eines der folgenden Enzyme codiert:
1.) Transkriptionsregulator der L-Methionin-Biosynthese (MetJ) (b3938, ECK3930),
2.) Glucose-6-phosphat-Isomerase (Pgi, EC 5.3.1.9) (b4025, ECK4017),
3.) Homoserinkinase (ThrB, EC 2.7.1.39) (b0003, ECK0003),
4.) S-Adenosylmethionin-Synthase (MetK, EC 2.5.1.6) (b2942, ECK2937),
5.) Dihydrodipicolinat-Synthase (DapA, EC 4.2.1.52) (b2478, ECK2474),
6.) Phosphoenolpyruvat-Carboxykinase (Pck, EC 4.1.1.49) (b3403, ECK3390),
7.) Formyltetrahydrofolat-Hydrolase (PurU, EC 3.5.1.10) (b1232, ECK1227),
8.) Pyruvatkinase II (PykA, EC 2.7.1.40) (b1854, ECK1855),
9.) Pyruvatkinase I (PykF, EC 2.7.1.40) (b1676, ECK1672),
10.) Untereinheit des L-Methionin-Transporters (MetQNI) (b0197, ECK0197),
11.) Untereinheit des L-Methionin-Transporters (MetQNI) (b0198, ECK0198),
12.) Untereinheit des L-Methionin-Transporters (MetQNI) (b0199, ECK0199),
13.) Desoxycytidin-5'-triphosphat-Deaminase (Dcd, EC 3.5.4.13) (b2065, ECK2059),
14.) putative N-Acyltransferase (YncA),
15.) regulatorische sRNA FnrS,
16.) Sigma-Faktor RpoS.

10. *E. coli*-Zelle nach einem der Ansprüche 4 bis 9, wobei die *E. coli*-Zelle relativ zu ihrem Wildtyp wenigstens eine Nukleinsäuresequenz überexprimiert, die für eines der folgenden Enzyme codiert:
1.) Anthranilat-Synthase (trpDE, EC 4.1.3.27), Anthranilat-Phosphoribosyltransferase (trpD, EC 2.4.2.18), Phosphoribosylanthranilat-Isomerase (trpC, EC 5.3.1.24), Indol-3-glycerinphosphat-Synthase (trpC, EC 4.1.1.48) und Tryptophan-Synthase (trpAB, EC 4.1.2.8 und 4.2.1.122),
2.) Phosphoglycerat-Dehydrogenase SerA (EC 1.1.1.95),
3.) 3-Phosphoserin-Phosphatase SerB (EC 3.1.3.3),
4.) 3-Phosphoserin/Phosphohydroxythreonin-Aminotransferase SerC (EC 2.6.1.52),
5.) L-Tyrosin-sensitive DHAP-Synthase (aroF, EC 2.5.1.54),
6.) L-Phenylalanin-Feedback-resistente DHAP-Synthase (aroG, EC 2.5.1.54),
7.) L-Tryptophan-sensitive DHAP-Synthase (aroH, EC 2.5.1.54),
8.) Phosphoenolpyruvat-Synthase ppsA (EC 2.7.9.2),
9.) Phosphoenolpyruvat-Carboxykinase pck (EC 4.1.1.49),
10.) Transketolase A tktA (EC 2.2.1.1),
11.) Transketolase B tktB (EC 2.2.1.1),
12.) Genprodukt des offenen Leserasters (ORF) yddG aus *E. coli.*

11. *E. coli*-Zelle nach einem der Ansprüche 4 bis 10, wobei die *E. coli*-Zelle relativ zu ihrem Wildtyp wenigstens eine Nukleinsäuresequenz in einem verringerten Ausmaß exprimiert, die für eines der folgenden Enzyme codiert:
1.) Tryptophanase (tnaA, EC 4.1.99.1),
2.) Repressor des trp-Operons (trpR),
3.) Chorismat-Mutase T oder Prephenat-Dehydrogenase (tyrA, EC 1.3.1.12),
4.) Chorismat-Mutase P oder Prephenat-Dehydrogenase (pheA, EC 4.2.1.51),
5.) Tryptophan-spezifisches Transportprotein (mtr),
6.) Tryptophan-Permease (tnaB),
7.) Transporter für aromatische Aminosäuren (aroP),
8.) L-Serin-Deaminase (sdaA, EC 4.3.1.17),
9.) Glucose-6-phosphat-Isomerase (pgi, EC 5.3.1.9),
10.) Tyrosin-Aminotransferase (tyrB),
11.) Repressor des glp-Regulons (glpR);
12.) Sigma-Faktor RpoS (rpoS).

12. Verfahren zur Herstellung von Methionin oder Tryptophan, umfassend den Schritt des Kultivierens einer *E*. *coli*-Zelle mit der in einem der Ansprüche 4 bis 11 angegebenen Bedeutung.

## Revendications

1. Procédé de préparation de méthionine ou de tryptophane, comprenant l'étape de culture d'une cellule de *E. coli,* qui surproduit déjà la méthionine ou le tryptophane et est en outre génétiquement modifiée par rapport à son type sauvage de telle manière qu'elle ait une activité ppGppase réduite par rapport à son type sauvage, dans lequel la cellule de *E. coli* présente une activité (p)ppGpp-synthétase qui est pratiquement inchangée par rapport à son type sauvage et dans lequel la ppGppase est au moins l'une choisie dans le groupe comprenant les séquences d'acides aminés SEQ ID NO: 2, SEQ ID NO: 4 et SEQ ID NO: 6.

2. Procédé selon la revendication 1, dans lequel l'activité dans la cellule de *E. coli* cultivée d'au moins une ppGppase du groupe comprenant les séquences d'acides aminés SEQ ID NO: 2, SEQ ID NO: 4 et SEQ ID NO: 6 est réduite en conséquence du fait que ladite ppGppase comporte au moins la modification suivante : une insertion des deux acides aminés His et Asn est présente entre Asp84 et Met85.

3. Procédé selon la revendication 1 ou 2, comprenant en outre l'étape de purification de l'acide aminé essentiel.

4. Cellule de *E. coli,* qui surproduit déjà la méthionine ou le tryptophane, dans laquelle l'activité d'au moins une ppGppase dans le groupe comprenant les séquences d'acides aminés SEQ ID NO: 2, SEQ ID NO: 4 et SEQ ID NO: 6 est réduite en conséquence du fait que ladite ppGppase comporte la modification suivante : une insertion des deux acides aminés His et Asn est présente entre Asp84 et Met85, et la cellule de *E. coli* ayant une activité (p)ppGpp-synthétase qui est pratiquement inchangée par rapport à son type sauvage.

5. Cellule de *E. coli* selon la revendication 4 dans laquelle l'activité d'au moins une ppGppase comprenant la séquence d'acides aminés SEQ ID NO: 2 est réduite en conséquence du fait que ladite ppGppase comporte les modifications suivantes : une insertion des deux acides aminés His et Asn est présente entre Asp84 et Met85 et la cellule de *E. coli* ayant une activité (p)ppGpp-synthétase qui est pratiquement inchangée par rapport à son type sauvage.

6. Cellule de *E. coli* selon la revendication 5, dans laquelle la ppGppase comporte en outre au moins une modification dans le groupe comprenant des substitutions aux acides aminés Gln9, Thr13, Tyr63, Arg109, Gln225, Cys245, Val248, Asn268, Ser270, Met280, His344, Pro436, Asn501, Gln505, His543, Ala546, Ser547, Ile548, His555, Gly556, His557, Pro559, Lys619, Thr621, Ala622, Thr627, Thr651, Ala669, Ala675, Thr698 et une insertion entre Glu343 et His344.

7. Cellule de *E. coli* selon l'une des revendications 4 à 6, dans laquelle la ppGppase comporte au moins une modification du groupe comportant les substitutions suivantes :
1.) substitution de Gln9 par un acide aminé choisi dans le groupe constitué de Leu, Ile et Val,
2.) substitution de Thr13 par un acide aminé choisi dans le groupe constitué de Lys, Arg, His, Gln et Asn,
3.) substitution de Tyr63 par un acide aminé choisi dans le groupe constitué de Lys, Arg et His,
4.) substitution de Arg109 par un acide aminé choisi dans le groupe constitué de Gln et Asn,
5.) substitution de Gln225 par un acide aminé choisi dans le groupe constitué de Ser, Ala et Thr,
6.) substitution de Cys245 par un acide aminé choisi dans le groupe constitué de Leu, Ile et Val,
7.) substitution de Val248 par un acide aminé choisi dans le groupe constitué de Lys, Arg et His,
8.) substitution de Asn268 par un acide aminé choisi dans le groupe constitué de Lys, Arg et His,
9.) substitution de Ser270 par un acide aminé choisi dans le groupe constitué de Ala, Leu, Ile et Val,
10.) substitution de Met280 par un acide aminé choisi dans le groupe constitué de Ser, Thr et Ala,
11.) insertion des acides aminés Lys et Glu entre les acides aminés Glu343 et His344,
12.) substitution de His344 par un acide aminé choisi dans le groupe constitué de Gln et Asn,
13.) substitution de Pro436 par un acide aminé choisi dans le groupe constitué de Ser, Ala et Thr,
14.) substitution de Asn501 par un acide aminé choisi dans le groupe constitué de Ser, Ala et Thr,
15.) substitution de Gln505 par un acide aminé choisi dans le groupe constitué de Pro, Ser, Ala et Thr,
16.) substitution de His543 par un acide aminé choisi dans le groupe constitué de Asn et Gln,
17.) substitution de Ala546 par un acide aminé choisi dans le groupe constitué de Asn et Gln,
18.) substitution de Ser547 par un acide aminé choisi dans le groupe constitué de Ala, Leu, Ile et Val,
19.) substitution de Ile548 par un acide aminé choisi dans le groupe constitué de Asn et Gln,
20.) substitution de His555 par un acide aminé choisi dans le groupe constitué de Leu, Ile et Val,
21.) substitution de glycine à la position 556 par Lys, Arg et His,
22.) substitution de His557 par un acide aminé choisi dans le groupe constitué de Asn et Gln,
23.) substitution de Pro559 par un acide aminé choisi dans le groupe constitué de Ser, Ala et Thr,
24.) substitution de Lys619 par un acide aminé choisi dans le groupe constitué de Asn et Gln,
25.) substitution de Thr621 par un acide aminé choisi dans le groupe constitué de Leu, Ile et Val,
26.) substitution de Ala622 par un acide aminé choisi dans le groupe constitué de Glu et Asp,
27.) substitution de Thr627 par un acide aminé choisi dans le groupe constitué de Ala et Gly,
28.) substitution de Thr651 par un acide aminé choisi dans le groupe constitué de Glu et Asp,
29.) substitution de Ala669 et/ou Ala675 par Thr,
30.) substitution de Thr698 par un acide aminé choisi dans le groupe constitué de Gln et Asn.

8. Cellule de *E. coli* selon l'une quelconque des revendications 4 à 7, la cellule de *E. coli* surexprimant, par rapport à son type sauvage, au moins une séquence d'acide nucléique qui code pour l'une quelconque des enzymes suivantes
1.) système de transport de thiosulfate/sulfate CysPUWA (EC 3.6.3.25),
2.) 3'-phosphoadénosine 5'-phosphosulfate réductase CysH (EC 1.8.4.8),
3.) sulfite réductase CysJI (EC 1.8.1.2),
4.) cystéine synthase A CysK (EC 2.5.1.47),
5.) cystéine synthase B CysM (EC 2.5.1.47),
6.) sérine acétyltransférase CysE (EC 2.3.1.30),
7.) système de clivage de glycine GcvTHP-Lpd (EC 2.1.2.10, EC 1.4.4.2, EC 1.8.1.4),
8.) lipoyle synthase LipA (EC 2.8.1.8),
9.) lipoyl-protéine ligase LipB (EC 2.3.1.181),
10.) phosphoglycérate déshydrogénase SerA (EC 1.1.1.95),
11.) 3-phosphosérine phosphatase SerB (EC 3.1.3.3),
12.) 3-phosphosérine/phosphohydroxythréonine aminotransférase SerC (EC 2.6.1.52),
13.) sérine hydroxyméthyltransférase GlyA (EC 2.1.2.1),
14.) aspartokinase I et homosérine déshydrogénase I ThrA (EC 2.7.2.4, EC 1.1.1.3),
15.) aspartate kinase LysC (EC 2.7.2.4),
16.) homosérine déshydrogénase Horn (EC 1.1.1.3),
17.) homosérine O-acétyltransférase MetX (EC 2.3.1.31),
18.) homosérine O-succinyltransférase MetA (EC 2.3.1.46),
19.) cystathionine gamma-synthase MetB (EC 2.5.1.48),
20.) C-S lyase AecD (EC 4.4.1.8, également appelée bêta-lyase),
21.) cystathionine bêta-lyase MetC (EC 4.4.1.8),
22.) homocystéine S-méthyltransférase B12-indépendante MetE (EC 2.1.1.14),
23.) homocystéine S-méthyltransférase B12-dépendante MetH (EC 2.1.1.13),
24.) méthylènetétrahydrofolate réductase MetF (EC 1.5.1.20),
25.) exportateur de L-méthionine BrnFE de *Corynebacterium glutamicum,*
26.) exportateur de valine YgaZFI *d'Escherichia coli* (b2682, b2683),
27.) transporteur putatif YjeFI *d'Escherichia coli* (b4141),
28.) pyridine nucléotide transhydrogénase PntAB (EC 1.6.1.2),
29.) O-succinylhomosérine sulfhydrylase MetZ (EC 2.5.1.48),
30.) phosphoénolpyruvate carboxylase Pyc (EC 4.1.1.31),
31.) thiosulfate sulfurtransférase RDL2p (EC 2.8.1.1),
32.) thiosulfate-thiol sulfurtransférase (EC 2.8.1.3),
33.) thiosulfate-dithiol sulfurtransférase (EC 2.8.1.5).

9. Cellule de *E. coli* selon l'une quelconque des revendications 4 à 8, la cellule de *E. coli* exprimant à une échelle réduite, par rapport à son type sauvage, au moins une séquence d'acide nucléique qui code pour l'une quelconque des enzymes suivantes :
1.) régulateur transcriptionnel de biosynthèse de L-méthionine (MetJ) (b3938, ECK3930),
2.) glucose-6-phosphate isomérase (Pgi, EC 5.3.1.9) (b4025, ECK4017),
3.) homosérine kinase (ThrB, EC 2.7.1.39) (b0003, ECK0003),
4.) S-adénosylméthionine synthase (MetK, EC 2.5.1.6) (b2942, ECK2937),
5.) dihydrodipicolinate synthase (DapA, EC 4.2.1.52) (b2478, ECK2474),
6.) phosphoénolpyruvate carboxykinase (Pck, EC 4.1.1.49) (b3403, ECK3390),
7.) formyltétrahydrofolate hydrolase (PurU), EC 3.5.1.10) (b1232, ECK1227),
8.) pyruvate kinase II (PykA, EC 2.7.1.40) (b1854, ECK1855),
9.) pyruvate kinase I (PykF, EC 2.7.1.40) (b1676, ECK1672),
10.) sous-unité de transporteur de L-méthionine (MetQNI) (b0197, ECK0197),
11.) sous-unité de transporteur de L-méthionine (MetQNI) (b0198, ECK0198),
12.) sous-unité de transporteur de L-méthionine (MetQNI) (b0199, ECK0199),
13.) désoxycytidine 5'-triphosphate désaminase (Dcd, EC 3.5.4.13) (b2065, ECK2059),
14.) N-acyltransférase putative (YncA),
15.) ARNs régulateur FnrS,
16.) facteur sigma RpoS.

10. Cellule de *E. coli* selon l'une quelconque des revendications 4 à 9, la cellule de *E. coli* surexprimant, par rapport à son type sauvage, au moins une séquence d'acide nucléique qui code pour l'une quelconque des enzymes suivantes :
1.) anthranilate synthase (trpDE, EC 4.1.3.27), anthranilate phosphoribosyl-transférase (trpD, EC 2.4.2.18), phosphoribosylanthranilate isomérase (trpC, EC 5.3.1.24), indole-3-glycérol-phosphate synthase (trpC, EC 4.1.1.48) et tryptophane synthase (trpAB, EC 4.1.2.8 et 4.2.1.122),
2.) phosphoglycérate déshydrogénase SerA (EC 1.1.1.95),
3.) 3-phosphosérine phosphatase SerB (EC 3.1.3.3),
4.) 3-phosphosérine/phosphohydroxythréonine aminotransférase SerC (EC 2.6.1.52),
5.) DHAP synthase sensible à la L-tyrosine (aroF, EC 2.5.1.54),
6.) DHAP synthase résistante à la rétroaction par la L-phénylalanine (aroG, EC 2.5.1.54),
7.) DHAP synthase sensible au L-tryptophane (aroH, EC 2.5.1.54),
8.) phosphoénolpyruvate synthase ppsA (EC 2.7.9.2),
9.) phosphoénolpyruvate carboxykinase pck (EC 4.1.1.49),
10.) transcétolase A tktA (EC 2.2.1.1),
11.) transcétolase B tktB (EC 2.2.1.1),
12.) produit génique du cadre ouvert de lecture (ORF) de *E. coli* yddG.

11. Cellule de *E. coli* selon l'une quelconque des revendications 4 à 10, la cellule de *E. coli* exprimant à une échelle réduite, par rapport à son type sauvage, au moins une séquence d'acide nucléique qui code pour l'une quelconque des enzymes suivantes :
1.) tryptophanase (tnaA, EC 4.1.99.1),
2.) répresseur de l'opéron trp (trpR),
3.) chorismate mutase T ou préphénate déshydrogénase (tyrA, EC 1.3.1.12),
4.) chorismate mutase P ou préphénate déshydrogénase (pheA, EC 4.2.1.51),
5.) protéine de transport spécifique du tryptophane (mtr),
6.) tryptophane perméase (tnaB),
7.) transporteur pour acides aminés aromatiques (aroP),
8.) L-sérine désaminase (sdaA, EC 4.3.1.17),
9.) glucose-6-phosphate isomérase (pgi, EC 5.3.1.9),
10.) tyrosine aminotransférase (tyrB),
11.) répresseur du régulon glp (glpR) ;
12.) facteur sigma RpoS (rpoS).

12. Procédé de préparation de méthionine ou de tryptophane, comprenant l'étape de culture d'une cellule de *E. coli* telle que définie dans l'une quelconque des revendications 4 à 11.
